# EUROPÄISCHE PATENTSCHRIFT

(11) **EP 1 380 263 B1**
(45) Veröffentlichungstag und Bekanntmachung des Hinweises auf die Patenterteilung: **29.08.2007**
(21) Anmeldenummer: 03015826.5
(22) Anmeldetag: 10.07.2003
(51) Int. Cl.: A61B 6/03, G01T 1/29

(54) **Verfahren und Vorrichtung zur Bestimmung der Ist-Position einer Struktur eines Untersuchungsobjektes**
Process and device for measuring the actual position of the structure of an object to be examined
Procédé et dispositif pour la mesure de la position instantanée d'une structure d'un objet à examiner

(30) Priorität: 12.07.2002 DE 10231896; 08.07.2003 EP 03015337
(43) Veröffentlichungstag der Anmeldung: 14.01.2004
(60) Teilanmeldung: 07005650.2
(73) Patentinhaber: MYCRONA Gesellschaft für innovative Messtechnik mbH, 66793 Saarwellingen (DE)
(72) Erfinder: Schroeder, Mario, 66346 Püttlingen (DE); Schmidt, Wolfram, 66125 Saarbrücken (DE)
(74) Vertreter: Mierswa, Klaus

(56) Entgegenhaltungen:
- CH-A- 691 655
- DE-A- 19 819 928
- US-A1- 2002 085 681
- FULTON R R ET AL: "Correction for head movements in positron emission tomography using an optical motion tracking system" NUCLEAR SCIENCE SYMPOSIUM CONFERENCE RECORD, 2000 IEEE LYON, FRANCE 15-20 OCT. 2000, PISCATAWAY, NJ, USA,IEEE, US, 25. Oktober 2000 (2000-10-25), Seiten 1758-1762, XP010556640 ISBN: 0-7803-6503-8

## Beschreibung

### Technisches Gebiet:

Die Erfindung bezieht sich auf Verfahren und eine Vorrichtung zur Bestimmung der Ist-Position einer Struktur eines Untersuchungsobjektes in einem Koordinatensystem.

### Stand der Technik:

Computer-Tomographen dienen zur Erstellung von dreidimensionalen Bildern, nämlich so genannten CT-Bildern, von Objekten, z.B. Werkstücken oder menschlichen Körpern oder Körperteilen, wobei diese Bilder auch innere Strukturen des Objektes zeigen.

Aus der DE 198 19 928 A1 ist ein Verfahren zur Bestimmung der Lage einer Schicht bei einem Schichtbilder erzeugenden Abbildungssystems bekannt, bei welchem folgende Schritte ausgeführt werden: die Position des Abbildungssystems wird mit einem Positionsmeßsystem gemessen, und die Lage der Schicht wird aus der gemessenen Position und aus gespeicherten Kalibrierdaten berechnet.

Aus der US 5,186,174 ist ein Verfahren zur Darstellung der Lage eines Instruments auf einem Tomogramm bekannt, wobei sich das Instrument in einem Objekt befindet, welches durch das Tomogramm dargestellt ist. Hierzu werden drei Referenzpunkte auf dem Objekt festgelegt.

In der Computer-Tomographie-Technik, abgekürzt CT-Technik, werden mit Hilfe von Röntgenstrahlen Aufnahmen eines Objekts oder eines Teils desselben aus vielen verschiedenen Richtungen hergestellt, d.h. das Objekt wird nacheinander aus vielen verschiedenen Richtungen durchleuchtet. Ein Computer-Tomograph, im Folgenden mit CT abgekürzt, verfügt daher über eine Röntgenquelle und einen zweidimensional ortsauflösenden Detektor, z.B. CCD-Matrix, welcher für die von der Röntgenquelle abgegebene Strahlung empfindlich ist. Die Röntgenquelle gibt Strahlung von typischerweise z.B. 450 keV ab. Zwischen Röntgenquelle und Detektor wird das Objekt angeordnet und schrittweise gegenüber der Röntgenquelle bzw. dem Schirm gedreht, oder es wird umgekehrt der CT schrittweise um das Objekt gedreht, welches in diesem Fall in Ruhe bleibt.

In den Fällen, in welchen nicht der CT, sondern das Objekt gedreht wird, verfügt der CT in der Regel über einen Objektträgertisch, auf welchem das Objekt angeordnet ist. Der Objektträgertisch ist so verfahrbar, daß sich das Objekt in den Strahlengang der Röntgenstrahlung einbringen läßt. Ferner ist der Objektträgertisch rotierbar, so daß das Objekt zur Erstellung des CT-Bildes gedreht werden kann. Typischerweise wird das Objekt z.B. in Schritten von je 0,9° gedreht, so daß 400 Rotationsschritte eine volle Umdrehung des Objektes ergeben. Auf diese Weise ist der CT imstande, ein bestimmtes Volumen, welches entweder das gesamte Objekt oder einen Teil desselben enthält, zu erfassen

Für jede der so durchlaufenen Rotationsstellungen des Objektes wird mit dem Detektor ein zweidimensionales Durchstrahlungs-Röntgenbild des Objektes aufgenommen. Auf diesen zweidimensionalen Bildern erscheint das Objekt größer als es in Wirklichkeit ist, da seine Größe auf dem Bild einer zentrischen Projektion des Objektes auf die Detektorfläche entspricht, wobei die Röntgenquelle das Projektionszentrum ist.

Aus der so gewonnenen Vielzahl von zweidimensionalen Einzelbildern wird mit Hilfe eines Computers rechnerisch ein dreidimensionales digitales Bild des vom CT erfaßten Volumens, d.h. des Objekts oder eines Teils desselben, erstellt, welches auch vollständig im Objekt eingeschlossene innere Strukturen desselben zeigt, sofern diese einen gegenüber ihrer Umgebung abweichenden Absorptionskoeffizienten für die eingestrahlte Röntgenstrahlung aufweisen, was z.B. für Hohlräume, wie etwa Bohrungen, der Fall ist.

Nach jeder vollen Umdrehung des Objekts kann der Objektträger mit dem Objekt um eine bestimmte Strecke translatorisch verschoben und der oben erläuterte Vorgang erneut durchgeführt werden.

Ein derartiger handelsüblicher CT ist z.B. in dem Prospekt "RayScan 3D-X-Ray-Computed Tomgraphy", PRO-RS-A-E000 11/01, der Firma Hans Wälischmiller GmbH, D-88677 Markdorf, beschrieben.

Mit derartigen Computer-Tomographen können innere Strukturen von Objekten untersucht werden, z.B. Bohrungen in Werkstücken. Um auf diese Weise eine Struktur des Objektes zu untersuchen, kann zunächst ein CT-Bild des ganzen Objektes einschließlich der interessierenden Struktur erstellt werden.

Nachteilig hierbei ist, dass das Bild der Struktur in der Regel nur einen kleinen Teil des Bildfeldes des CT einnimmt, da die mit dem CT erreichbare relative räumliche Auflösung begrenzt ist. Dies ist insbesondere auf den endlichen Durchmesser der Austrittspupille der Röntgenquelle und auf die begrenzte Zahl der Pixel der CCD-Matrix zurückzuführen; typisch wird eine relative laterale Auflösung von z.B. 1:4000 erreicht.

Eine Struktur des Objektes, deren Ausdehnung z.B. 1% der Objektgröße beträgt, wird daher in diesem Fall nur mit einer relativen Auflösung von 1:40 abgebildet, was in den meisten Fällen für eine detaillierte Vermessung der Struktur unzureichend ist.

Daher kann das CT-Bild des Objektes dazu herangezogen werden, um den Ort der Struktur innerhalb des Objektes in Bezug auf das Koordinatensystem des CT zu ermitteln und eine zweite Computer-Tomographie des Objektes durchzuführen und dabei den CT so zu steuern, dass nur die nähere Umgebung der Struktur von dem CT erfaßt wird und von dieser Umgebung ein zweites CT-Bild, mit erhöhtem Vergrößerungsfaktor, erstellt wird. Hierdurch wird die relative Auflösung des Bildes der Struktur gesteigert, d.h. es werden mehr Details der Struktur sichtbar.

Auch hiermit sind jedoch Nachteile verbunden. Beispielsweise ist es aufwendig, den Ort innerhalb des Objektes aus dem ersten CT-Bild zu bestimmen. Darüber hinaus ist eine derartige Lokalisierung der Struktur ungenau, da nicht nur die Struktur selbst, sondern auch die Oberfläche des Objektes nur mit der begrenzten Auflösung des CT erfaßt werden, wodurch sich die entsprechenden Meßunsicherheiten vergrößern.

Ferner wird zur Erstellung des zweiten CT-Bildes zusätzliche Zeit benötigt. Dieser zusätzliche Zeitaufwand stellt angesichts der sehr hohen Betriebskosten eines CT einen erheblichen Kostenfaktor bei der Vermessung der Struktur dar.

Darüber hinaus wird das Objekt zur Erstellung des zweiten CT-Bildes erneut mit einer bestimmten Dosis an ionisierender Strahlung beaufschlagt. Dies ist insbesondere dann nachteilig bzw. problematisch, wenn das Objekt aus lebender biologischer Materie besteht. Auch auf tote Materie kann die erneute Strahlenbelastung nachteilig einwirken. Ionisierende Strahlung kann beispielsweise Alterung, Umwandlung, Verfärbung oder Zersetzung von Kunststoffen auslösen, auf Kristallstrukturen verändernd einwirken oder elektronische Bausteine zerstören.

### Technische Aufgabe:

Der Erfindung liegt daher die Aufgabe zu Grunde, ein Verfahren und eine Vorrichtung anzugeben, welche eine Lokalisierung und Untersuchung einer Struktur eines Objektes mit verringertem Zeitaufwand, erhöhter Genauigkeit und reduzierter Strahlenbelastung des Objektes ermöglichen.

Diese Aufgabe wird erfindungsgemäß gelöst durch ein Verfahren zur Bestimmung der Ist-Position einer Struktur eines Untersuchungsobjektes in einem Koordinatensystem, wobei ein Computer-Tomograph in Anwendung der CT-Technik mit einem auf den Computer-Tomographen bezogenen ersten Koordinatensystem, CT-Koordinatensystem, und ein Koordinatenmeßgerät, welches entweder ein taktiles oder optisches Koordinatenmeßgerät oder ein Multisensor-Koordinatenmeßgerät oder ein Ultraschall-Koordinatenmeßgerät ist, mit einem auf dieses bezogenen zweiten Koordinatensystem, MG-Koordinatensystem, verwendet werden, wobei
a) im MG-Koordinatensystem die Koordinaten des Untersuchungsobjekts bestimmt werden,
b) eine Soll-Position der Struktur innerhalb des Untersuchungsobjektes vorgegeben wird,
c) die Soll-Position nach Ausführung der Schritte a) und b) im MG-Koordinatensystem bestimmt wird,
d) und das Untersuchungsobjekt unter Verwendung des Ergebnisses von Schritt c) so positioniert wird, dass die Soll-Position der Struktur innerhalb des vom Computer-Tomographen erfassten Volumens zu liegen kommt,
oder
aa) im CT-Koordinatensystem die Koordinaten des Untersuchungsobjekts bestimmt werden,
bb) eine Soll-Position der Struktur innerhalb des Untersuchungsobjektes vorgegeben wird,
cc) die Soll-Position nach Ausführung der Schritte aa) und bb) im CT-Koordinatensystem bestimmt wird,
dd) und das Untersuchungsobjekt unter Verwendung des Ergebnisses von Schritt cc) so positioniert wird, dass die Soll-Position der Struktur innerhalb des vom Koordinatenmeßgerät erfaßbaren Bereichs zu liegen kommt.

Somit betrifft die Erfindung ein Verfahren zur Bestimmung der Ist-Position einer Struktur eines Untersuchungsobjektes in einem Koordinatensystem, wobei ein Computer-Tomograph in Anwendung der CT-Technik mit einem auf den Computer-Tomographen bezogenen ersten Koordinatensystem, CT-Koordinatensystem, und ein Koordinatenmeßgerät, welches entweder ein taktiles oder optisches Koordinatenmeßgerät oder ein Multisensor-Koordinatenmeßgerät oder ein Ultraschall-Koordinatenmeßgerät ist, mit einem auf dieses bezogenen zweiten Koordinatensystem, MG-Koordinatensystem, verwendet werden, wobei
a) im MG-Koordinatensystem die Koordinaten des Untersuchungsobjekts bestimmt werden,
b) eine Soll-Position der Struktur innerhalb des Untersuchungsobjektes vorgegeben wird,
c) die Soll-Position nach Ausführung der Schritte a) und b) im MG-Koordinatensystem bestimmt wird,
d) und das Untersuchungsobjekt unter Verwendung des Ergebnisses von Schritt c) so positioniert wird, dass die Soll-Position der Struktur innerhalb des vom Computer-Tomographen erfassten Volumens zu liegen kommt.

Somit betrifft die Erfindung des Weiteren ein Verfahren zur Bestimmung der Ist-Position einer Struktur eines Untersuchungsobjektes in einem Koordinatensystem, wobei ein Computer-Tomograph in Anwendung der CT-Technik mit einem auf den Computer-Tomographen bezogenen ersten Koordinatensystem, CT-Koordinatensystem, und ein Koordinatenmeßgerät, welches entweder ein taktiles oder optisches Koordinatenmeßgerät oder ein Multisensor-Koordinatenmeßgerät oder ein Ultraschall-Koordinatenmeßgerät ist, mit einem auf dieses bezogenen zweiten Koordinatensystem, MG-Koordinatensystem, verwendet werden, wobei
**a) im CT-Koordinatensystem die Koordinaten des** Untersuchungsobjekts bestimmt werden,
b) eine Soll-Position der Struktur innerhalb des Untersuchungsobjektes vorgegeben wird,
c) die Soll-Position nach Ausführung der Schritte a) und b) im CT-Koordinatensystem bestimmt wird,
d) und das Untersuchungsobjekt unter Verwendung des Ergebnisses von Schritt c) so positioniert wird, dass die Soll-Position der Struktur innerhalb des vom Koordinatenmeßgerät erfaßbaren Bereichs zu liegen kommt.

Die Aufgabe wird ferner gelöst durch eine Vorrichtung zur Bestimmung der Ist-Position einer Struktur eines Untersuchungsobjektes in einem Koordinatensystem, mit einem Computer-Tomograph mit einem auf den Computer-Tomographen bezogenen ersten Koordinatensystem, CT-Koordinatensystem, und einem Koordinatenmeßgerät, welches entweder ein taktiles oder optisches Koordinatenmeßgerät oder ein Multisensor-Koordinatenmeßgerät oder ein Ultraschall-Koordinatenmeßgerät ist, mit einem auf dieses bezogenen zweiten Koordinatensystem, MG-Koordinatensystem, wobei im MG-Koordinatensystem die Koordinaten des Untersuchungsobjekts bestimmbar sind und eine Soll-Position der Struktur innerhalb des Untersuchungsobjektes vorgegeben ist, so dass die Soll-Position im MG-Koordinatensystem bestimmbar ist und bei in Bezug auf mindestens drei ausgewählte nicht kollineare Punkte des Untersuchungsobjekts vorgegebener Soll-Position der Struktur das Untersuchungsobjekt mit Hilfe des Koordinatenmeßgerätes so positionierbar ist, dass mindestens ein Teil des Untersuchungsobjekts in dem vom Computer-Tomographen erfaßten Volumen liegt und dieser Teil des Untersuchungsobjekts die Soll-Position der Struktur enthält, wodurch die Soll-Position der Struktur innerhalb des vom Computer-Tomographen erfassten Volumens zu liegen kommt, wobei der Computer-Tomograph und das Multisensor-Koordinatenmeßgerät zu einer einzigen Vorrichtung integriert sind.

Somit ist eine Vorrichtung zur Bestimmung der Ist-Position einer Struktur eines Untersuchungsobjektes in einem Koordinatensystem vorhanden, mit einem Computer-Tomograph mit einem auf den Computer-Tomographen bezogenen ersten Koordinatensystem, CT-Koordinatensystem, und einem Koordinatenmeßgerät, welches entweder ein taktiles oder optisches Koordinatenmeßgerät oder ein Multisensor-Koordinatenmeßgerät oder ein Ultraschall-Koordinatenmeßgerät ist, mit einem auf dieses bezogenen zweiten Koordinatensystem, MG-Koordinatensystem, wobei im MG-Koordinatensystem die Koordinaten des Untersuchungsobjekts bestimmbar sind und eine Soll-Position der Struktur innerhalb des Untersuchungsobjektes vorgegeben ist, so dass die Soll-Position im MG-Koordinatensystem bestimmbar ist und das Untersuchungsobjekt so positionierbar ist, dass die Soll-Position der Struktur innerhalb des vom Computer-Tomographen erfassten Volumens zu liegen kommt, wobei der Computer-Tomograph und das Multisensor-Koordinatenmeßgerät zu einer einzigen Vorrichtung integriert sind.

Das erste Koordinatensystem ist also das CT-Koordinatensystem, welches auf den Computer-Tomographen bezogen ist. Das zweite Koordinatensystem ist das MG-Koordinatensystem; dieses ist auf das Koordinatenmeßgerät bezogen.

Bei dem erfindungsgemäßen Verfahren zur Bestimmung der Ist-Position einer Struktur eines Untersuchungsobjektes in einem Koordinatensystem werden also ein Computer-Tomograph mit einem auf diesen bezogenen ersten Koordinatensystem, CT-Koordinatensystem, und ein Koordinatenmeßgerät, welches entweder ein taktiles oder optisches Koordinatenmeßgerät oder ein Multisensor-Koordinatenmeßgerät oder ein Ultraschall-Koordinatenmeßgerät ist, mit einem auf dieses bezogenen zweiten Koordinatensystem, MG-Koordinatensystem, verwendet.

Die erfindungsgemäße Vorrichtung zur Bestimmung der Ist-Position und der Form einer Struktur eines Untersuchungsobjektes in einem Koordinatensystem umfaßt einen Computer-Tomographen mit einem auf diesen bezogenen ersten Koordinatensystem, CT-Koordinatensystem, und ein Koordinatenmeßgerät, welches entweder ein taktiles oder optisches Koordinatenmeßgerät oder ein Multisensor-Koordinatenmeßgerät oder ein Ultraschall-Koordinatenmeßgerät ist, mit einem auf dieses bezogenen zweiten Koordinatensystem, MG-Koordinatensystem.

Gemäß einer Variante des Verfahrens wird bei in Bezug auf mindestens drei ausgewählte nicht kollineare Punkte des Untersuchungsobjekts vorgegebener Soll-Position der Struktur das Untersuchungsobjekt mit Hilfe des Koordinatenmeßgerätes so positioniert, dass mindestens ein Teil des Untersuchungsobjekts in dem vom Computer-Tomographen erfaßten Volumen liegt und dieser Teil des Untersuchungsobjekts die Soll-Position der Struktur enthält.

Erfindungsgemäß ist bei in Bezug auf mindestens drei ausgewählte nicht kollineare Punkte des Untersuchungsobjekts vorgegebener Soll-Position der Struktur das Untersuchungsobjekt mit Hilfe des Koordinatenmeßgerätes so positionierbar, dass mindestens ein Teil des Untersuchungsobjekts in dem vom Computer-Tomographen erfaßten Volumen liegt und dieser Teil des Untersuchungsobjekts die Soll-Position der Struktur enthält.

Die ausgewählten Punkte können sich insbesondere auf der Oberfläche des Untersuchungsobjekts befinden. Die ausgewählten Punkte können markiert werden, beispielsweise mittels Farbe, um ihre Erfassung im MG-Koordinatensystem zu erleichtern. Eine andere Möglichkeit besteht darin, als ausgewählte Punkte solche zu verwenden, welche auf Grund der Geometrie bzw. der Gestalt des Untersuchungsobjekts ausgezeichnet sind, z.B. Eckpunkte.

In einer Variante des Verfahrens wird bei vorgegebener maximaler Abweichung der Soll-Position von der Ist-Position der Struktur des Untersuchungsobjekts dasselbe mit Hilfe des Koordinatenmeßgerätes so positioniert, dass sowohl die Soll-Position als auch die Ist-Position der Struktur in dem vom Computer-Tomographen erfaßten Volumen liegen.

Gemäß einer Ausführungsform der erfindungsgemäßen Vorrichtung ist daher bei vorgegebener maximaler Abweichung der Soll-Position von der Ist-Position der Struktur des Untersuchungsobjekts dasselbe mit Hilfe des Koordinatenmeßgerätes so positionierbar, dass sowohl die Soll-Position als auch die Ist-Position der Struktur in dem vom Computer-Tomographen erfaßten Volumen liegen.

Die Ist-Position der Struktur kann z.B. infolge von Fertigungstoleranzen von der Soll-Position abweichen. In der Praxis ist es oftmals möglich, die maximal zu erwartende oder mögliche Abweichung Ist-Position von der Soll-Position der Struktur anzugeben.

Gemäß einer Variante des Verfahrens ist die Ist-Position um höchstens eine vorgegebene Toleranzabweichung von der Soll-Position verschieden, so dass sich die Ist-Position innerhalb eines Toleranzvolumens befindet, dessen Rand um höchstens die Toleranzabweichung von der Soll-Position entfernt ist, wobei das Untersuchungsobjekt mit Hilfe des Koordinatenmeßgerätes so positioniert wird, dass das Toleranzvolumen vollständig in dem vom Computer-Tomographen erfaßten Volumen liegt.

In einer Ausführungsform der erfindungsgemäßen Vorrichtung ist die Ist-Position um höchstens eine vorgegebene Toleranzabweichung von der Soll-Position verschieden, so dass sich die Ist-Position innerhalb eines Toleranzvolumens befindet, dessen Rand um höchstens die Toleranzabweichung von der Soll-Position entfernt ist, wobei das Untersuchungsobjekt mit Hilfe des Koordinatenmeßgerätes so positionierbar ist, dass das Toleranzvolumen vollständig in dem vom Computer-Tomographen erfaßten Volumen liegt.

Insbesondere kann das Toleranzvolumen eine Kugel, Toleranzkugel, sein, deren Mittelpunkt mit der Soll-Positionen zusammenfällt und deren Radius durch den Betrag der maximalen Abweichung der Soll-Position von der Ist-Position der Struktur vorgegeben ist.

Gemäß einer Variante des Verfahrens wird das Untersuchungsobjekt mit Hilfe des Koordinatenmeßgerätes so positioniert, dass das vom Computer-Tomographen erfaßte Volumen höchstens den x-fachen Rauminhalt der Toleranzkugel bzw. des Toleranzvolumens besitzt, wobei x eine vorgebbare Zahl vorzugsweise größer als 1 ist.

In einer Variante der erfindungsgemäßen Vorrichtung ist das Untersuchungsobjekt mit Hilfe des Koordinatenmeßgerätes so positionierbar, dass das vom Computer-Tomographen erfaßte Volumen höchstens den x-fachen Rauminhalt der Toleranzkugel bzw. des Toleranzvolumens besitzt, wobei x eine vorgebbare Zahl vorzugsweise größer als 1 ist.

Beispielsweise kann das Untersuchungsobjekt mit Hilfe des Koordinatenmeßgerätes so positioniert werden, dass das vom Computer-Tomographen erfaßte Volumen höchstens den doppelten Rauminhalt der Toleranzkugel bzw. des Toleranzvolumens besitzt, was bedeutet, dass die Zahl x in diesem Beispiel gleich 2 ist.

Gemäß einem anderen Beispiel wird das Untersuchungsobjekt mit Hilfe des Koordinatenmeßgerätes so positioniert, dass das vom Computer-Tomographen erfaßte Volumen höchstens den Vierfachen Rauminhalt der Toleranzkugel bzw. des Toleranzvolumens besitzt, was bedeutet, dass die Zahl x in diesem Beispiel gleich 4 ist.

Gemäß einer Variante wird
- bei in Bezug auf mindestens drei ausgewählte nicht kollineare Punkte des Untersuchungsobjekts (1) vorgegebener Soll-Position der Struktur das Untersuchungsobjekt (1) mit Hilfe des Computer-Tomographen so positioniert, dass mindestens ein Teil des Untersuchungsobjekts (1) in dem vom Koordinatenmeßgerät erfaßbaren Bereich liegt und dieser Teil des Untersuchungsobjekts (1) die Soll-Position der Struktur enthält,
- bei vorgegebener maximaler Abweichung der Soll-Position von der Ist-Position der Struktur des Untersuchungsobjekts (1) dasselbe mit Hilfe des Computer-Tomographen so positioniert, dass sowohl die Soll-Position als auch die Ist-Position der Struktur in dem vom Koordinatenmeßgerät erfaßbaren Bereich liegen, wobei die Ist-Position um höchstens eine vorgegebene Toleranzabweichung von der Soll-Position verschieden ist, so dass sich die Ist-Position innerhalb eines Toleranzbereichs befindet, dessen Rand um höchstens die Toleranzabweichung von der Soll-Position entfernt ist, und
- das Untersuchungsobjekt (1) mit Hilfe des Computer-Tomographen so positioniert, dass der Toleranzbereich vollständig in dem vom Koordinatenmeßgerät erfaßbaren Bereich liegt.

Die relative Lage und die relative Orientierung des CT-Koordinatensystems gegenüber dem MG-Koordinatensystem können vorgegeben sein oder durch Einmessung ermittelt werden. Gemäß einer Ausführungsform sind daher die relative Lage und die relative Orientierung des CT-Koordinatensystems gegenüber dem MG-Koordinatensystem vorgegeben oder durch Einmessung ermittelbar.

Gemäß einer Variante werden folgende Schritte ausgeführt:
(i) mittels des Koordinatenmeßgerätes wird die Lage der mindestens drei ausgewählten Punkte des Untersuchungsobjektes bezüglich des MG-Koordinatensystems bestimmt,
(ii) die auf das MG-Koordinatensystem bezogene Soll-Position der Struktur wird mit Hilfe der im Schritt (i) erzielten Meßergebnisse berechnet, und
(iii) die Soll-Position der Struktur wird vom MG-Koordinatensystem auf das CT-Koordinatensystem umgerechnet, so dass anschließend die Lage der Soll-Position im CT-Koordinatensystem bekannt ist.

Gemäß einer Ausführungsform ist
(i) mittels des Koordinatenmeßgerätes die Lage der mindestens drei ausgewählten Punkte des Untersuchungsobjektes bezüglich des MG-Koordinatensystems bestimmbar,
(ii) die auf das MG-Koordinatensystem bezogene Soll-Position der Struktur mit Hilfe der gemäß (i) erzielten Meßergebnisse berechenbar, und
(iii) die Soll-Position der Struktur vom MG-Koordinatensystem auf das CT-Koordinatensystem umrechenbar, so dass die Lage der Soll-Position im CT-Koordinatensystem bestimmbar ist.

Gemäß einer Variante wird das Untersuchungsobjekt gegenüber dem Computer-Tomographen unter Verwendung der gemäß Schritt (iii) erhaltenen, auf das CT-Koordinatensystem bezogenen Soll-Position der Struktur mittels einer Verfahreinrichtung so gesteuert, dass sich das Toleranzvolumen und daher auch die Struktur in dem vom Computer-Tomographen erfaßten Volumen befindet.

Gemäß einer Ausführungsform der Erfindung ist daher das Untersuchungsobjekt gegenüber dem Computer-Tomographen unter Verwendung der gemäß (iii) erhaltenen, auf das CT-Koordinatensystem bezogenen Soll-Position der Struktur mittels einer Verfahreinrichtung so steuerbar, dass sich das Toleranzvolumen und daher auch die Struktur in dem vom Computer-Tomographen erfaßten Volumen befindet.

Gemäß einer Variante wird mit Hilfe des Computer-Tomographen ein dreidimensionales digitales CT-Bild des Toleranzvolumens einschließlich der Struktur erstellt und als CT-Datensatz gespeichert und die Ist-Position der Struktur im CT-Koordinatensystem aus dem CT-Datensatz bestimmt.

Gemäß einer Ausführungsform ist daher mit Hilfe des Computer-Tomographen ein dreidimensionales digitales CT-Bild des Toleranzvolumens einschließlich der Struktur erstellbar und als CT-Datensatz speicherbar und die Ist-Position der Struktur im CT-Koordinatensystem aus dem CT-Datensatz bestimmbar.

Gemäß einer Variante wird
(i) mittels des Computer-Tomographen die Lage der mindestens drei ausgewählten Punkte des Untersuchungsobjektes bezüglich des CT-Koordinatensystems bestimmt,
(ii) die auf das CT-Koordinatensystem bezogene Soll-Position der Struktur mit Hilfe der im Schritt (i) erzielten Meßergebnisse berechnet,
(iii) die Soll-Position der Struktur vom CT-Koordinatensystem auf das MG-Koordinatensystem umgerechnet, so dass anschließend die Lage der Soll-Position im CT-Koordinatensystem bekannt ist,
(iv) das Untersuchungsobjekt gegenüber dem Koordinatenmeßgerät unter Verwendung der gemäß Schritt (iii) erhaltenen, auf das MG-Koordinatensystem bezogenen Soll-Position der Struktur mittels einer Verfahreinrichtung so gesteuert, dass sich das Toleranzvolumen und daher auch die Struktur in dem vom Koordinatenmeßgerät erfaßbaren Bereich befindet, und
(v) mit Hilfe des Koordinatenmeßgerätes ein dreidimensionales digitales Bild des Toleranzbereichs einschließlich der Struktur erstellt und als MG-Datensatz gespeichert und die Ist-Position der Struktur im MG-Koordinatensystem aus dem MG-Datensatz bestimmt.

Gemäß einer bevorzugten Variante des erfindungsgemäßen Verfahrens wird als Computer-Tomograph ein solcher verwendet, welcher eine Röntgenquelle und einen zweidimensional ortsauflösenden Detektor aufweist, welcher eine aktive Detektorfläche besitzt, die für die von der Röntgenquelle abgegebene Röntgenstrahlung empfindlich ist, wobei das Bildfeld des Computer-Tomographen durch die Größe der aktiven Sensor- oder Detektorfläche gegeben ist, die Soll-Position der Struktur in Bezug auf mindestens drei ausgewählte, nicht kollineare Punkte des Untersuchungsobjektes vorgegeben ist und die Ist-Position um höchstens eine Toleranzabweichung von der Soll-Position verschieden ist, so dass sich die Ist-Position innerhalb eines z.B. kugelförmigen Toleranzvolumens befindet, dessen Rand um höchstens die Toleranzabweichung von der Soll-Position entfernt ist. Die relative Lage und die relative Orientierung des CT-Koordinatensystems gegenüber dem MG-Koordinatensystem sind hierbei entweder bereits bekannt oder werden durch Einmessung ermittelt.

Das Koordinatenmeßgerät kann ein taktiles, d.h. auf mechanischer Abtastung beruhendes, oder ein optisches Koordinatenmeßgerät oder ein z.B. lasergestütztes Multisensor-Koordinatenmeßgerät oder ein Ultraschall-Koordinatenmeßgerät sein. Das Koordinatenmeßgerät kann also insbesondere ein solches sein, welches nicht imstande ist, innere Strukturen des Untersuchungsobjektes zu erfassen.

Gemäß dieser Variante werden folgende Schritte ausgeführt:
a) Mittels des Koordinatenmeßgerätes wird die Lage der mindestens drei ausgewählten Punkte des Untersuchungsobjektes bezüglich des MG-Koordinatensystems bestimmt,
b) die auf das MG-Koordinatensystem bezogene Soll-Position der Struktur wird mit Hilfe der im Schritt a) erzielten Meßergebnisse berechnet,
c) die Soll-Position der Struktur wird vom MG-Koordinatensystem auf das CT-Koordinatensystem umgerechnet, so dass die Lage derselben im CT-Koordinatensystem bekannt ist,
d) die Relativposition des Untersuchungsobjektes bezüglich des Computer-Tomographen wird unter Verwendung der gemäß Schritt c) erhaltenen, auf das CT-Koordinatensystem bezogenen Soll-Position der Struktur mittels einer Verfahreinrichtung so gesteuert, dass sich das Toleranzvolumen und daher auch die Struktur in demjenigen Volumen befindet, welches der Computer-Tomograph zu erfassen imstande ist,
e) mit Hilfe des Computer-Tomographen wird ein dreidimensionales digitales CT-Bild des Toleranzvolumens einschließlich der Struktur erstellt und als CT-Datensatz gespeichert, und
f) die Ist-Position der Struktur im CT-Koordinatensystem wird aus dem CT-Datensatz bestimmt.

Dies bedeutet, dass der CT mit Hilfe der durch das Koordinatenmeßgerät durchgeführten Messungen, der relativen Lage und der relativen Orientierung von CT-Koordinatensystem und MG-Koordinatensystem vorteilhafterweise so gesteuert werden kann, dass sich die Struktur von vornherein in dem vom CT erfaßten und abgebildeten Bereich des Untersuchungsobjektes befindet.

Gemäß einer bevorzugten Ausführungsform der erfindungsgemäßen Vorrichtung weist der Computer-Tomograph eine Röntgenquelle und einen zweidimensional ortsauflösenden Detektor auf, welcher eine aktive Detektorfläche besitzt, die für die von der Röntgenquelle abgegebene Röntgenstrahlung empfindlich ist, wobei das Bildfeld des Computer-Tomographen durch die Größe der aktiven Detektorfläche gegeben ist, die Soll-Position der Struktur in Bezug auf mindestens drei ausgewählte, nicht kollineare Punkte des Untersuchungsobjektes vorgegeben ist und die Ist-Position um höchstens eine Toleranzabweichung von der Soll-Position verschieden ist, so dass sich die Ist-Position innerhalb eines z.B. kugelförmigen Toleranzvolumens befindet, dessen Rand um höchstens die Toleranzabweichung von der Soll-Position entfernt ist, und die relative Lage und die relative Orientierung des CT-Koordinatensystems gegenüber dem MG-Koordinatensystem bekannt oder durch Einmessung ermittelbar sind, wobei
a) mittels des Koordinatenmeßgerätes die Lage der mindestens drei ausgewählten Punkte des Untersuchungsobjektes bezüglich des MG-Koordinatensystems bestimmbar ist,
b) hieraus die auf das MG-Koordinatensystem bezogene Soll-Position der Struktur berechenbar ist,
c) die Soll-Position der Struktur vom MG-Koordinatensystem auf das CT-Koordinatensystem umrechenbar ist, so dass die Lage derselben im CT-Koordinatensystem bestimmbar ist,
d) die Relativposition des Untersuchungsobjektes bezüglich des Computer-Tomographen unter Verwendung der auf das CT-Koordinatensystem bezogenen Soll-Position der Struktur mittels einer Verfahreinrichtung so steuerbar ist, dass sich das Toleranzvolumen und daher auch die Struktur in demjenigen Volumen befindet, welches der Computer-Tomograph zu erfassen imstande ist, und
e) der Computer-Tomograph imstande ist, ein dreidimensionales digitales CT-Bild des Toleranzvolumens einschließlich der Struktur zu erstellen und als CT-Datensatz zu speichern,
so dass die Ist-Position sowie die Form der Struktur im CT-Koordinatensystem aus dem CT-Datensatz bestimmbar sind.

Mit aktiver Detektorfläche ist diejenige Fläche des Detektors gemeint, die zur Registrierung der Röntgenstrahlung von der Röntgenquelle nutzbar ist. Unter dem Bildfeld des Computer-Tomographen wird hierbei die Ausdehnung der Projektion des vom Computer-Tomographen erfaßten Volumens auf die Ebene der Detektorfläche verstanden.

Somit ist erfindungsgemäß die Möglichkeit geschaffen, den Computer-Tomographen, abgekürzt CT, bei so hoher Vergrößerung zu betreiben, dass er nicht mehr das ganze Untersuchungsobjekt synchron abbilden kann, und hierbei die Relativposition zwischen CT und Untersuchungsobjekt von vornherein so zu steuern, dass vorteilhafterweise immer das Toleranzvolumen und somit auch die Struktur vom CT erfaßt wird.

Zur Bestimmung der auf das MG-Koordinatensystem bezogenen Soll-Position der Struktur ist die Vermessung von mindestens drei ausgewählten Punkten des Untersuchungsobjektes bezüglich des MG-Koordinatensystems erforderlich; werden mehr als drei Punkte auf die genannte Weise vermessen, so lassen sich die zusätzlichen Meßergebnisse vorteilhaft zur Verringerung des mittleren Fehlers und damit zur Erhöhung der erzielten Genauigkeit verwenden.

Ein Vorteil der Erfindung besteht darin, dass die äußere Form von Werkstücken oder anderen Objekten z.B. in der Serienproduktion in vielen Fällen routinemäßig ohnehin mit einem taktilen oder optischen Koordinatenmeßgerät oder mit einem Multisensor-Koordinatenmeßgerät vermessen wird, z.B. zum Zweck der Produktionsüberwachung. Die Ergebnisse des Schrittes a) stehen in diesen Fällen von vornherein zur Verfügung, so dass der Schritt a) keinen zusätzlichen Aufwand mit sich bringt.

Das Toleranzvolumen kann insbesondere rotationssymmetrisch, d.h. eine Toleranzkugel sein, so dass deren Radius durch die Toleranzabweichung und deren Mittelpunkt durch die Soll-Position gegeben ist.

Gemäß einer zweckmäßigen Variante der Erfindung wird der Computer-Tomograph im Verfahrensschritt d) so gesteuert, dass sich das Zentrum des Toleranzvolumens im wesentlichen im Zentrum des von dem Computer-Tomographen erfaßbaren Volumens befindet. Bevorzugt ist daher der Computer-Tomograph so steuerbar, dass sich das Zentrum des Toleranzvolumens im wesentlichen im Zentrum des von dem Computer-Tomographen erfaßbaren Volumens befindet.

Dies bedeutet, dass der CT erfindungsgemäß so gesteuert wird, dass sich die Soll-Position der Struktur vorteilhafterweise von vornherein im Zentrum des vom CT erfaßten und abgebildeten Bereichs des Untersuchungsobjektes befindet; die Erfindung ermöglicht von vornherein eine "Zentrierung" der Soll-Position der Struktur in dem vom CT erfaßbaren Bereich.

Gemäß einer Variante wird der Computer-Tomograph so gesteuert, dass bei zentrischer Projektion des Toleranzvolumens mit der Röntgenquelle als Projektionszentrum das Bildfeld durch die Projektion des Toleranzvolumens auf den Detektor vollständig ausgefüllt wird, so dass die relative laterale Auflösungsfähigkeit der aktiven Detektorfläche vollständig zur Erfassung des Toleranzvolumens ausgenutzt wird. In diesem Fall ist der Computer-Tomograph so steuerbar, dass bei zentrischer Projektion des Toleranzvolumens mit der Röntgenquelle als Projektionszentrum das Bildfeld durch die Projektion des Toleranzvolumens auf den Detektor vollständig ausgefüllt ist.

Gemäß einer anderen bevorzugten Variante wird der Computer-Tomograph so gesteuert, dass bei zentrischer Projektion des Toleranzvolumens mit der Röntgenquelle als Projektionszentrum der kleinste Durchmesser der Projektion des Toleranzvolumens auf den Detektor und der kleinste Durchmesser des Bildfeldes des Computer-Tomographen im wesentlichen gleich groß sind, oder der größte Durchmesser der Projektion des Toleranzvolumens auf den Detektor und der größte Durchmesser des Bildfeldes des Computer-Tomographen im wesentlichen gleich groß sind.

Gemäß einer weiteren Variante wird der Computer-Tomograph so gesteuert, dass bei zentrischer Projektion des Toleranzvolumens mit der Röntgenquelle als Projektionszentrum der größte Durchmesser der Projektion des Toleranzvolumens auf den Detektor und der kleinste Durchmesser des Bildfeldes des Computer-Tomographen im wesentlichen gleich groß sind.

In einer Variante ist der Computer-Tomograph so steuerbar, dass bei zentrischer Projektion des Toleranzvolumens mit der Röntgenquelle als Projektionszentrum der kleinste Durchmesser der Projektion des Toleranzvolumens auf den Detektor und der kleinste Durchmesser des Bildfeldes des Computer-Tomographen im wesentlichen gleich groß sind, oder der größte Durchmesser der Projektion des Toleranzvolumens auf den Detektor und der größte Durchmesser des Bildfeldes des Computer-Tomographen im wesentlichen gleich groß sind.

Gemäß einer weiteren Variante ist der Computer-Tomograph so steuerbar, dass bei zentrischer Projektion des Toleranzvolumens mit der Röntgenquelle als Projektionszentrum der größte Durchmesser der Projektion des Toleranzvolumens auf den Detektor und der kleinste Durchmesser des Bildfeldes des Computer-Tomographen im wesentlichen gleich groß sind.

Auf diese Weise kann der zur Erstellung des CT-Bildes verwendete Vergrößerungsfaktor so an die Abmessungen des Bildfeldes und an die Größe des Tolerenzvolumens angepaßt werden, dass die Projektion des Tolerenzvolumens im wesentlichen mit der Größe des Bildfeldes übereinstimmt. Der Vergrößerungsfaktor kann auf diese Weise vorteilhaft so gewählt werden, dass er der größtmögliche ist, bei welchem das Tolerenanzvolumens gerade noch vollständig als Ganzes vom CT erfaßbar ist, so dass sich die Struktur vorteilhafterweise von vornherein im dem vom CT erfaßten und abgebildeten Bereich befindet. Auch bei hohem Vergrößerungsfaktor besteht daher keine Gefahr, dass sich die Struktur außerhalb des vom CT erfaßbaren Bereiches befindet. Hierbei wird die relative laterale Auflösungsfähigkeit der aktiven Detektorfläche weitgehend zur Erfassung des Toleranzvolumens ausgenutzt.

Das Untersuchungsobjekt kann z.B. eine Kfz-Einspritzdüse mit einer typischen Länge von 50 mm und einem typischen mittleren Durchmesser von 25 mm sein. Die zu untersuchende Struktur kann z.B. in die Einspritzdüse eingebrachte Bohrung von einigen Millimetern Länge sein.

Die Soll-Position der Struktur ist bei solchen Strukturen, welche gezielt an Objekten angebracht werden, wie beispielsweise die Bohrung einer Einspritzdüse, ebenfalls von vornherein bekannt. In vielen Fällen erfolgt die Produktion derartiger Objekte mit großer Präzision, d.h. mit sehr geringen Toleranzen zwischen Soll- und Ist-Position, so dass das Toleranzvolumen sehr klein ist und der Vergrößerungsfaktor vorteilhafterweise entsprechend hoch gewählt werden kann.

Gemäß einer Variante wird
A) das Untersuchungsobjekt zur Erstellung des CT-Bildes schrittweise um eine Rotationsachse gedreht,
B) für jede der so durchlaufenen Rotationsstellungen des Untersuchungsobjektes mit dem Detektor ein zweidimensionales Durchstrahlungs-Röntgenbild des Untersuchungsobjektes aufgenommen, und
C) aus den so erhaltenen zweidimensionalen Durchstrahlungs-Röntgenbildern das dreidimensionale CT-Bild erstellt.

Gemäß einer weiteren Variante wird
D) das Untersuchungsobjekt nach Ausführung der Schritte A) und B) um eine bestimmte Strecke vorzugsweise in einer Richtung parallel zur Rotationsachse translatorisch verschoben und danach erneut schrittweise um die Rotationsachse gedreht;
E) für jede der im Schritt D) durchlaufenen Rotationsstellungen des Untersuchungosbjektes mit dem Detektor wiederum ein zweidimensionales Durchstrahlungs-Röntgenbild des Untersuchungsobjektes aufgenommen, und
F) aus den im Schritt E) erhaltenen zweidimensionalen Durchstrahlungs-Röntgenbildern ein weiteres dreidimensionales CT-Bild erstellt.

Die Schritte D) bis F) können mehrmals wiederholt werden.

Gemäß einer anderen Variante der Erfindung wird das Untersuchungsobjekt zur Erstellung eines dreidimensionalen CT-Bildes nicht nur schrittweise um einen vorgebbaren Drehwinkel gedreht, sondern nach jedem dieser Rotationsschritte um eine bestimmte Strecke translatorisch verschoben, so dass die nicht auf der Drehachse liegenden Punkte des Untersuchungsobjekts eine im wesentlichen spiralförmige Bahn beschreiben.

Aus dem CT-Bild oder dem CT-Datensatz kann zusätzlich zur Lage der Struktur auch die Form der Struktur ermittelt werden. Beispielsweise kann die Form der Begrenzungsfläche einer kleinen Bohrung in einer Einspritzdüse mit Hilfe der Erfindung sehr genau vermessen werden. Die Soll-Lage der Struktur kann dabei auf einen ausgewählten Punkt der Struktur bezogen sein, wobei die Koordinaten weiterer Punkte der Struktur relativ zu diesem Punkt der Struktur aus dem CT-Bild bestimmt werden können. Die so ermittelten Koordinaten von Punkten der Struktur können z.B. zur Parametrisierung der Form der Struktur herangezogen werden.

Gemäß einer anderen Variante wird aus dem CT-Bild oder dem CT-Datensatz anstelle der Lage der Struktur die Form der Struktur ermittelt.

Die relative Lage und die relative Orientierung zwischen MG-Koordinatensystem und CT-Koordinatensystem läßt sich bestimmen, indem die Lage von mindestens drei, vorzugsweise von mindestens vier ausgewählten Aufpunkten eines Kalibrierobjektes sowohl mit dem Computer-Tomographen im CT-Koordinatensystem als auch mit dem Koordinaten-Meßgerät im MG-Koordinatensystem bestimmt wird. Aus dem Vergleich der so erhaltenen Ergebnisse können die relative Lage und die relative Orientierung des CT-Koordinatensystems gegenüber MG-Koordinatensystem, z.B. eine Transformationsmatrix zur Überführung dieser beiden Koordinatensystem ineinander, ermittelt werden. Werden mehr als drei Aufpunkte auf die genannte Weise eingemessen, so ist die genannte Koordinatentransformation mathematisch überbestimmt; die redundanten Ergebnisse lassen sich jedoch vorteilhaft zur Verringerung des mittleren Fehlers und damit zur Erhöhung der erzielten Genauigkeit kombinieren.

Das Untersuchungsobjekt und das Kalibrierobjekt können selbstverständlich identisch sein. Ebenso können die ausgewählten Punkte des Untersuchungsobjektes mit den zu gegenseitigen Einmessung der beiden Koordinatensysteme verwendeten Aufpunkten zusammenfallen.

Gemäß einer bevorzugten Variante sind der Computer-Tomograph und das Multisensor-Koordinatenmeßgerät zu einer einzigen Vorrichtung integriert.

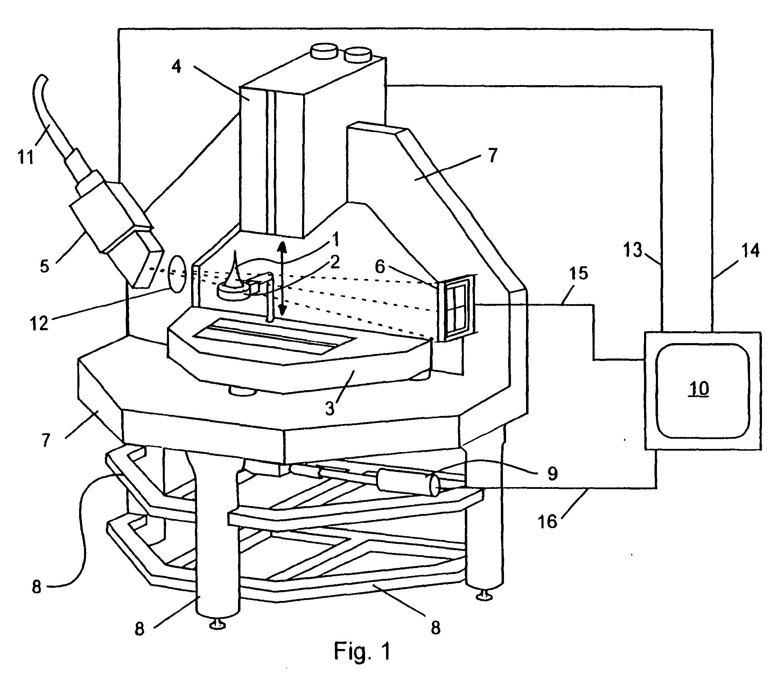

Figur 1 zeigt schematisch ein Ausführungsbeispiel einer erfindungsgemäßen Vorrichtung zur Bestimmung der Ist-Position und der Form einer Struktur eines Untersuchungsobjektes 1 in einem Koordinatensystem.

Die Vorrichtung von Fig. 1 dient zur Bestimmung der Ist-Position und der Form einer Struktur eines Untersuchungsobjektes in einem Koordinatensystem. Die Vorrichtung umfaßt einen Computer-Tomographen, im folgenden als CT bezeichnet, mit einem auf diesen bezogenen Koordinatensystem, im folgenden als CT-Koordinatensystem bezeichnet, und ein Multisensor-Koordinatenmeßgerät mit einem auf dieses bezogenen Koordinatensystem, im folgenden als MG-Koordinatensystem bezeichnet.

Der CT umfaßt eine Röntgenquelle 5 und einen zweidimensional ortsauflösenden Detektor 6. Die Soll-Position der Struktur ist in Bezug auf mindestens drei ausgewählte, nicht kollineare Punkte des Untersuchungsobjektes 1 vorgegeben. Die Ist-Position der Struktur ist um höchstens eine Toleranzabweichung von der Soll-Position verschieden, so dass sich die Ist-Position innerhalb eines z.B. kugelförmigen Toleranzvolumens befindet, dessen Rand um höchstens die Toleranzabweichung von der Soll-Position entfernt ist. Die relative Lage und die relative Orientierung des CT-Koordinatensystems gegenüber dem MG-Koordinatensystem sind bekannt oder durch Einmessung ermittelbar.

Mittels des Koodinatenmeßgerätes ist die Lage der mindestens drei ausgewählten Punkte des Untersuchungsobjektes 1 bezüglich des MG-Koordinatensystems bestimmbar. Hieraus ist die auf das MG-Koordinatensystem bezogene Soll-Position der Struktur berechenbar. Die Soll-Position der Struktur ist vom MG-Koordinatensystem auf das CT-Koordinatensystem umrechenbar, so dass die Lage derselben im CT-Koordinatensystem bestimmbar ist.

Die Relativposition des Untersuchungsobjektes 1 bezüglich des CT ist unter Verwendung der auf das CT-Koordinatensystem bezogenen Soll-Position der Struktur mittels einer Verfahreinrichtung 3 so steuerbar, dass sich das Toleranzvolumen und daher auch die Struktur in demjenigen Volumen befindet, welches der Computer-Tomograph zu erfassen imstande ist.

Der CT ist imstande, ein dreidimensionales digitales CT-Bild des Toleranzvolumens einschließlich der Struktur zu erstellen und als CT-Datensatz zu speichern, so dass die Ist-Position sowie die Form der Struktur im CT-Koordinatensystem aus dem CT-Datensatz bestimmbar sind.

Die Vorrichtung von Fig. 1 umfaßt einen Computer-Tomographen, im Folgenden mit CT abgekürzt, mit einer Röntgenquelle 5 und einem zweidimensional ortsauflösenden Detektor, z.B. eine CCD-Matrix 6, welche für die von der Röntgenquelle 5 abgegebene harte Röntgenstrahlung von typischerweise z.B. 450 keV empfindlich ist. Die Vorrichtung von Fig. 1 umfaßt ferner eine Sensor-Kamera-Einheit 4, welche oberhalb des Untersuchungsobjektes 1 angeordnet ist und einen mechanischen Taster, einen Lasertaster sowie zwei Kameras umfaßt und Teil eines Multisensor-Koordinatenmeßgerätes ist.

Das Untersuchungsobjekt 1 befindet sich auf einem Rotationstisch 2, welcher schrittweise rotierbar und seinerseits an einem Verfahrtisch 3 angeordnet ist. Der Rotationstisch 2 ist ferner gegenüber dem Verfahrtisch 3 aufwärts und abwärts translatorisch verfahrbar, was in Fig. 1 durch einen senkrechten Doppelpfeil angedeutet ist.

Der Verfahrtisch verfügt über einen Antrieb 9 und ist zweidimensional translatorisch verfahrbar, nämlich in der zu dem Doppelpfeil von Fig. 1 senkrechten Ebene. Die Röntgenquelle 5, die CCD-Matrix 6, die Sensor-Kamera-Einheit 4 sowie der Verfahrtisch 3 sind an einem gemeinsamen Montagerahmen 7 angeordnet, welcher auf einem nivellierbaren Unterbau 8 ruht. Durch Verfahren des Verfahrtisches 3 in Richtung auf die Röntgenquelle 5 wird der Vergrößerungsfaktor erhöht; durch Verfahren des Verfahrtisches 3 in die umgekehrte Richtung wird der Vergrößerungsfaktor verringert.

Das Untersuchungsobjekt 1 ist zwischen Röntgenquelle 5 und CCD-Matrix 6 angeordnet und wird zur Erstellung eines CT-Bildes mit der Röntgenstrahlung der Röntgenquelle 5 durchstrahlt, danach durch Drehen des Rotationstisches 2 um z.B. 0,9° gedreht und durch Verfahren des Verfahrtisches 3 um eine bestimmte Strecke translatorisch verschoben und erneut durchstrahlt, usw.

Für jede der so durchlaufenden Stellungen des Untersuchungsbjektes 1 wird mit der CCD-Matrix 6 ein zweidimensionales Durchstrahlungs-Röntgenbild des Untersuchungsobjektes 1 aufgenommen und aus der so gewonnenen Vielzahl von zweidimensionalen Einzelbildern wird ein dreidimensionales digitales Bild des vom CT erfaßten Bereiches des Untersuchungsobjekts 1 berechnet.

Diese Berechnung wird durch einen Berechnungs- und Steuerungscomputer 10 ausgeführt, welcher zugleich zur Steuerung des Rotationstisches 2, des Verfahrtisches 3, der Sensor-Kamera-Einheit 4 und der Röntgenquelle 5 dient und zu diesem Zweck über Leitungen 13, 14, 15, 16 mit den genannten Komponenten verbunden ist. Die Röntgenquelle wird durch ein Anschlußkabel 11 mit elektrischer Energie versorgt und ist imstande, einen das Untersuchungsobjekt 1 erfassenden Röntgenstrahlenkegel 12 auszusenden.

Der CT umfaßt die Röngenquelle 5, die CCD-Matrix 6, den Berechnungs- und Steuerungscomputer 10, den Rotationstisch 2, den Verfahrtisch 3 mit seinem Antrieb 9, den Montagerahmen 7 und seinen Unterbau 8.

Das Multisensor-Koordinatenmeßgerät umfaßt die Sensor-Kamera-Einheit 4, den Berechnungs- und Steuerungscomputer 10, den Rotationstisch 2, den Verfahrtisch 3 mit seinem Antrieb 9, den Montagerahmen 7 und seinen Unterbau 8.

Der Berechnungs- und Steuerungscomputer 10, der Rotationstisch 2, der Verfahrtisch 3 mit seinem Antrieb 9, der Montagerahmen 7 und sein Unterbau 8 gehören demnach sowohl zum CT als auch zum Multisensor-Koordinatenmeßgerät. In der Vorrichtung von Figur 1 sind somit erfindungsgemäß der CT und das Multisensor-Koordinatenmeßgerät zu einer einzigen Vorrichtung integriert.

Die gewerbliche Anwendbarkeit der Erfindung besteht darin, dass dieselbe in der zerstörungsfreien Prüfung und Überwachung von Gegenständen, insbesondere von Serienteilen, anwendbar ist. Ebenso ist die Erfindung in der Medizintechnik sowie zerstörungsfreien Werkstoffprüfung anwendbar zum Orten und Vermessen von inneren Strukturen. Die besondere Nützlichkeit der Erfindung besteht darin, dass eingeschlossene Strukturen eines Objektes, zum Beispiel Lunker oder Hohlräume, ohne Zerstörung oder Öffnen des Objektes geortet und gegenüber dem Stand der Technik auf Anhieb mit hoher Genauigkeit vermessen werden können.

### Liste der Bezugszeichen:

- 1: Untersuchungsobjekt
- 2: Rotiertisch
- 3: Verfahrtisch
- 4: Sensor-Kamera-Einheit
- 5: Röntgenquelle
- 6: CCD-Matrix
- 7: Montagerahmen
- 8: Unterbau
- 9: Antrieb für den Verfahrtisch
- 10: Berechnungs- und Steuerungscomputer
- 11: Anschlußkabel der Röntgenquelle
- 12: Röntgenstrahlenkegel aus der Röntgenquelle
- 13-16: Leitungen

## Patentansprüche

1. Verfahren zur Bestimmung der Ist-Position einer Struktur eines Untersuchungsobjektes (1) in einem Koordinatensystem, wobei ein Computer-Tomograph in Anwendung der CT-Technik mit einem auf den Computer-Tomographen bezogenen ersten Koordinatensystem, CT-Koordinatensystem, und ein Koordinatenmeßgerät, welches entweder ein taktiles oder optisches Koordinatenmeßgerät oder ein Multisensor-Koordinatenmeßgerät oder ein Ultraschall-Koordinatenmeßgerät ist, mit einem auf dieses bezogenen zweiten Koordinatensystem, MG-Koordinatensystem, verwendet werden, wobei
a) im MG-Koordinatensystem die Koordinaten des Untersuchungsobjekts (1) bestimmt werden,
b) eine Soll-Position der Struktur innerhalb des Untersuchungsobjektes (1) vorgegeben wird,
c) die Soll-Position nach Ausführung der Schritte a) und b) im MG-Koordinatensystem bestimmt wird,
d) und das Untersuchungsobjekt (1) unter Verwendung des Ergebnisses von Schritt c) so positioniert wird, dass die Soll-Position der Struktur innerhalb des vom Computer-Tomographen erfassten Volumens zu liegen kommt,
oder
aa) im CT-Koordinatensystem die Koordinaten des Untersuchungsobjekts (1) bestimmt werden,
bb) eine Soll-Position der Struktur innerhalb des Untersuchungsobjektes (1) vorgegeben wird,
cc) die Soll-Position nach Ausführung der Schritte aa) und bb) im CT-Koordinatensystem bestimmt wird,
dd) und das Untersuchungsobjekt (1) unter Verwendung des Ergebnisses von Schritt cc) so positioniert wird, dass die Soll-Position der Struktur innerhalb des vom Koordinatenmeßgerät erfaßbaren Bereichs zu liegen kommt.

2. Verfahren nach Anspruch 1,
wobei bei in Bezug auf mindestens drei ausgewählte nicht kollineare Punkte des Untersuchungsobjekts (1) vorgegebener Soll-Position der Struktur das Untersuchungsobjekt mit Hilfe des Koordinatenmeßgerätes so positioniert wird, dass mindestens ein Teil des Untersuchungsobjekts (1) in dem vom Computer-Tomographen erfaßten Volumen liegt und dieser Teil des Untersuchungsobjekts (1) die Soll-Position der Struktur enthält.

3. Verfahren nach Anspruch 1 oder 2,
wobei bei vorgegebener maximaler Abweichung der Soll-Position von der Ist-Position der Struktur des Untersuchungsobjekts (1) dasselbe mit Hilfe des Koordinatenmeßgerätes so positioniert wird, dass sowohl die Soll-Position als auch die Ist-Position der Struktur in dem vom Computer-Tomographen erfaßten Volumen liegen.

4. Verfahren nach Anspruch 3, wobei
- die Ist-Position um höchstens eine vorgegebene Toleranzabweichung von der Soll-Position verschieden ist, so dass sich die Ist-Position innerhalb eines Toleranzvolumens befindet, dessen Rand um höchstens die Toleranzabweichung von der Soll-Position entfernt ist, und
- das Untersuchungsobjekt mit Hilfe des Koordinatenmeßgerätes so positioniert wird, dass das Toleranzvolumen vollständig in dem vom Computer-Tomographen erfaßten Volumen liegt.

5. Verfahren nach Anspruch 4,
wobei das Toleranzvolumen eine Kugel, Toleranzkugel, ist, deren Mittelpunkt mit der Soll-Positionen zusammenfällt und deren Radius durch den Betrag der maximalen Abweichung der Soll-Position von der Ist-Position der Struktur vorgegeben ist.

6. Verfahren nach Anspruch 4 oder 5,
wobei das Untersuchungsobjekt mit Hilfe des Koordinatenmeßgerätes so positioniert wird, dass das vom Computer-Tomographen erfaßte Volumen höchstens den x-fachen Rauminhalt der Toleranzkugel bzw. des Toleranzvolumens besitzt, wobei x eine vorgebbare Zahl vorzugsweise größer als 1 ist.

7. Verfahren nach Anspruch 1, wobei
- bei in Bezug auf mindestens drei ausgewählte nicht kollineare Punkte des Untersuchungsobjekts (1) vorgegebener Soll-Position der Struktur das Untersuchungsobjekt (1) mit Hilfe des Computer-Tomographen so positioniert wird, dass mindestens ein Teil des Untersuchungsobjekts (1) in dem vom Koordinatenmeßgerät erfaßbaren Bereich liegt und dieser Teil des Untersuchungsobjekts (1) die Soll-Position der Struktur enthält,
- bei vorgegebener maximaler Abweichung der Soll-Position von der Ist-Position der Struktur des Untersuchungsobjekts (1) dasselbe mit Hilfe des Computer-Tomographen so positioniert wird, dass sowohl die Soll-Position als auch die Ist-Position der Struktur in dem vom Koordinatenmeßgerät erfaßbaren Bereich liegen,
- die Ist-Position um höchstens eine vorgegebene Toleranzabweichung von der Soll-Position verschieden ist, so dass sich die Ist-Position innerhalb eines Toleranzbereichs befindet, dessen Rand um höchstens die Toleranzabweichung von der Soll-Position entfernt ist, und
- das Untersuchungsobjekt (1) mit Hilfe des Computer-Tomographen so positioniert wird, dass der Toleranzbereich vollständig in dem vom Koordinatenmeßgerät erfaßbaren Bereich liegt.

8. Verfahren nach einem der Ansprüche 1 bis 7,
wobei die relative Lage und die relative Orientierung des CT-Koordinatensystems gegenüber dem MG-Koordinatensystem vorgegeben sind oder durch Einmessung ermittelt werden.

9. Verfahren nach Anspruch 2 und 8, wobei
(i) mittels des Koordinatenmeßgerätes die Lage der mindestens drei ausgewählten Punkte des Untersuchungsobjektes (1) bezüglich des MG-Koordinatensystems bestimmt wird,
(ii) die auf das MG-Koordinatensystem bezogene Soll-Position der Struktur mit Hilfe der im Schritt (i) erzielten Meßergebnisse berechnet wird, und
(iii) die Soll-Position der Struktur vom MG-Koordinatensystem auf das CT-Koordinatensystem umgerechnet wird, so dass anschließend die Lage der Soll-Position im CT-Koordinatensystem bekannt ist.

10. Verfahren nach Anspruch 9, wobei
das Untersuchungsobjekt (1) gegenüber dem Computer-Tomographen unter Verwendung der gemäß Schritt (iii) erhaltenen, auf das CT-Koordinatensystem bezogenen Soll-Position der Struktur mittels einer Verfahreinrichtung (3) so gesteuert wird, dass sich das Toleranzvolumen und daher auch die Struktur in dem vom Computer-Tomographen erfaßten Volumen befindet.

11. Verfahren nach einem der Ansprüche 1 bis 10,
wobei mit Hilfe des Computer-Tomographen ein dreidimensionales digitales CT-Bild des Toleranzvolumens einschließlich der Struktur erstellt und als CT-Datensatz gespeichert und die Ist-Position der Struktur im CT-Koordinatensystem aus dem CT-Datensatz bestimmt wird.

12. Verfahren nach Anspruch 7, wobei
(i) mittels des Computer-Tomographen die Lage der mindestens drei ausgewählten Punkte des Untersuchungsobjektes (1) bezüglich des CT-Koordinatensystems bestimmt wird,
(ii) die auf das CT-Koordinatensystem bezogene Soll-Position der Struktur mit Hilfe der im Schritt (i) erzielten Meßergebnisse berechnet wird,
(iii) die Soll-Position der Struktur vom CT-Koordinatensystem auf das MG-Koordinatensystem umgerechnet wird, so dass anschließend die Lage der Soll-Position im CT-Koordinatensystem bekannt ist,
(iv) das Untersuchungsobjekt (1) gegenüber dem Koordinatenmeßgerät unter Verwendung der gemäß Schritt (iii) erhaltenen, auf das MG-Koordinatensystem bezogenen Soll-Position der Struktur mittels einer Verfahreinrichtung (3) so gesteuert wird, dass sich das Toleranzvolumen und daher auch die Struktur in dem vom Koordinatenmeßgerät erfaßbaren Bereich befindet, und
(v) mit Hilfe des Koordinatenmeßgerätes ein dreidimensionales digitales Bild des Toleranzbereichs einschließlich der Struktur erstellt und als MG-Datensatz gespeichert und die Ist-Position der Struktur im MG-Koordinatensystem aus dem MG-Datensatz bestimmt wird.

13. Verfahren nach Anspruch 1, wobei
- als Computer-Tomograph ein solcher verwendet wird, welcher eine Röntgenquelle (5) und einen zweidimensional ortsauflösenden Detektor (6) aufweist, welcher eine aktive Detektorfläche besitzt, die für die von der Röntgenquelle (5) abgegebene Röntgenstrahlung empfindlich ist, wobei das Bildfeld des Computer-Tomographen durch die Größe der aktiven Detektorfläche gegeben ist,
- die Soll-Position der Struktur in Bezug auf mindestens drei ausgewählte, nicht kollineare Punkte des Untersuchungsobjektes (1) vorgegeben ist und die Ist-Position um höchstens eine Toleranzabweichung von der Soll-Position verschieden ist, so dass sich die Ist-Position innerhalb eines z.B. kugelförmigen Toleranzvolumens befindet, dessen Rand um höchstens die Toleranzabweichung von der Soll-Position entfernt ist, und
- die relative Lage und die relative Orientierung des CT-Koordinatensystems gegenüber dem MG-Koordinatensystem bekannt sind oder durch Einmessung ermittelt werden,
und folgende Schritte ausgeführt werden:
a) Mittels des Koordinatenmeßgerätes wird die Lage der mindestens drei ausgewählten Punkte des Untersuchungsobjektes (1) bezüglich des MG-Koordinatensystems bestimmt,
b) die auf das MG-Koordinatensystem bezogene Soll-Position der Struktur wird mit Hilfe der im Schritt a) erzielten Meßergebnisse berechnet,
c) die Soll-Position der Struktur wird vom MG-Koordinatensystem auf das CT-Koordinatensystem umgerechnet, so dass die Lage derselben im CT-Koordinatensystem bekannt ist,
d) die Relativposition des Untersuchungsobjektes (1) bezüglich des Computer-Tomographen wird unter Verwendung der gemäß Schritt c) erhaltenen, auf das CT-Koordinatensystem bezogenen Soll-Position der Struktur mittels einer Verfahreinrichtung (3) so gesteuert, dass sich das Toleranzvolumen und daher auch die Struktur in demjenigen Volumen befindet, welches der Computer-Tomograph zu erfassen imstande ist,
e) mit Hilfe des Computer-Tomographen wird ein dreidimensionales digitales CT-Bild des Toleranzvolumens einschließlich der Struktur erstellt und als CT-Datensatz gespeichert, und
f) die Ist-Position der Struktur im CT-Koordinatensystem wird aus dem CT-Datensatz bestimmt.

14. Verfahren nach Anspruch 13,
wobei das Toleranzvolumen eine Toleranzkugel ist, so dass deren Radius durch die Toleranzabweichung und deren Mittelpunkt durch die Soll-Position gegeben ist.

15. Verfahren nach Anspruch 13 oder 14,
wobei der Computer-Tomograph im Verfahrensschritt d) so gesteuert wird, dass sich das Zentrum des Toleranzvolumens im wesentlichen im Zentrum des von dem Computer-Tomographen erfaßbaren Volumens befindet.

16. Verfahren nach einem der Ansprüche 13 bis 15,
wobei der Computer-Tomograph so gesteuert wird, dass bei zentrischer Projektion des Toleranzvolumens mit der Röntgenquelle (5) als Projektionszentrum das Bildfeld durch die Projektion des Toleranzvolumens auf den Detektor vollständig ausgefüllt wird.

17. Verfahren nach einem der Ansprüche 15 oder 16,
wobei der Computer-Tomograph so gesteuert wird, dass bei zentrischer Projektion des Toleranzvolumens mit der Röntgenquelle (5) als Projektionszentrum
- der kleinste Durchmesser der Projektion des Toleranzvolumens auf den Detektor und der kleinste Durchmesser des Bildfeldes des Computer-Tomographen im wesentlichen gleich groß sind, oder
- der größte Durchmesser der Projektion des Toleranzvolumens auf den Detektor und der größte Durchmesser des Bildfeldes des Computer-Tomographen im wesentlichen gleich groß sind, oder
- der größte Durchmesser der Projektion des Toleranzvolumens auf den Detektor und der kleinste Durchmesser des Bildfeldes des Computer-Tomographen im wesentlichen gleich groß sind.

18. Verfahren nach einem der Ansprüche 1 bis 17,
wobei aus dem CT-Bild oder dem CT-Datensatz zusätzlich zur Lage der Struktur auch die Form der Struktur ermittelt wird.

19. Verfahren nach einem der Ansprüche 1 bis 17,
wobei aus dem CT-Bild oder dem CT-Datensatz anstelle der Lage der Struktur die Form der Struktur ermittelt wird.

20. Verfahren nach einem der Ansprüche 1 bis 19,
wobei die Lage von mindestens drei, vorzugsweise von mindestens vier ausgewählten Aufpunkten eines Kalibrierobjektes sowohl mit dem Computer-Tomographen im CT-Koordinatensystem als auch mit dem Koordinatenmeßgerät im MG-Koordinatensystem bestimmt wird und aus dem Vergleich der so erhaltenen Ergebnisse die relative Lage und die relative Orientierung des CT-Koordinatensystems gegenüber MG-Koordinatensystem ermittelt werden.

21. Verfahren nach Anspruch 20,
wobei das Untersuchungsobjekt (1) und das Kalibrierobjekt identisch sind.

22. Verfahren nach einem der Ansprüche 1 bis 21, wobei
A) das Untersuchungsobjekt (1) zur Erstellung des CT-Bildes schrittweise um eine Rotationsachse gedreht wird,
B) für jede der so durchlaufenen Rotationsstellungen des Untersuchungsobjektes (1) mit dem Detektor (6) ein zweidimensionales Durchstrahlungs-Röntgenbild des Untersuchungsobjektes (1) aufgenommen wird, und
C) aus den so erhaltenen zweidimensionalen Durchstrahlungs-Röntgenbildern das dreidimensionale CT-Bild erstellt wird.

23. Verfahren nach Anspruch 22, wobei
D) das Untersuchungsobjekt (1) nach Ausführung der Schritte A) und B) um eine bestimmte Strecke vorzugsweise in einer Richtung parallel zur Rotationsachse translatorisch verschoben und danach erneut schrittweise um die Rotationsachse gedreht wird;
E) für jede der im Schritt D) durchlaufenen Rotationsstellungen des Untersuchungosbjektes mit dem Detektor (6) wiederum ein zweidimensionales Durchstrahlungs-Röntgenbild des Untersuchungsobjektes (1) aufgenommen wird, und
F) aus den im Schritt E) erhaltenen zweidimensionalen Durchstrahlungs-Röntgenbildern ein weiteres dreidimensionales CT-Bild erstellt wird.

24. Vorrichtung zur Bestimmung der Ist-Position einer Struktur eines Untersuchungsobjektes (1) in einem Koordinatensystem, mit
- einem Computer-Tomograph mit einem auf den Computer-Tomographen bezogenen ersten Koordinatensystem, CT-Koordinatensysterri;
- und einem Koordinatenmeßgerät, welches entweder ein taktiles oder optisches Koordinatenmeßgerät oder ein Multisensor-Koordinatenmeßgerät oder ein Ultraschall-Koordinatenmeßgerät ist, mit einem auf dieses bezogenen zweiten Koordinatensystem, MG-Koordinatensystem,
wobei im MG-Koordinatensystem die Koordinaten des Untersuchungsobjekts (1) bestimmbar sind und eine Soll-Position der Struktur innerhalb des Untersuchungsobjektes (1) vorgegeben ist, so dass
- die Soll-Position im MG-Koordinatensystem bestimmbar ist
- und bei in Bezug auf mindestens drei ausgewählte nicht kollineare Punkte des Untersuchungsobjekts (1) vorgegebener Soll-Position der Struktur das Untersuchungsobjekt mit Hilfe des Koordinatenmeßgerätes so positionierbar ist, dass mindestens ein Teil des Untersuchungsobjekts (1) in dem vom Computer-Tomographen erfaßten Volumen liegt und dieser Teil des Untersuchungsobjekts (1) die Soll-Position der Struktur enthält, wodurch die Soll-Position der Struktur innerhalb des vom Computer-Tomographen erfassten Volumens zu liegen kommt,
wobei der Computer-Tomograph und das Multisensor-Koordinatenmeßgerät zu einer einzigen Vorrichtung integriert sind.

25. Vorrichtung nach Anspruch 24,
wobei bei vorgegebener maximaler Abweichung der Soll-Position von der Ist-Position der Struktur des Untersuchungsobjekts (1) dasselbe mit Hilfe des Koordinatenmeßgerätes so positionierbar ist, dass sowohl die Soll-Position als auch die Ist-Position der Struktur in dem vom Computer-Tomographen erfaßten Volumen liegen.

26. Vorrichtung nach Anspruch 25, wobei
- die Ist-Position um höchstens eine vorgegebene Toleranzabweichung von der Soll-Position verschieden ist, so dass sich die Ist-Position innerhalb eines Toleranzvolumens befindet, dessen Rand um höchstens die Toleranzabweichung von der Soll-Position entfernt ist, und
- das Untersuchungsobjekt mit Hilfe des Koordinatenmeßgerätes so positionierbar ist, dass das Toleranzvolumen vollständig in dem vom Computer-Tomographen erfaßten Volumen liegt.

27. Vorrichtung nach Anspruch 26,
wobei das Toleranzvolumen eine Kugel, Toleranzkugel, ist, deren Mittelpunkt mit der Soll-Positionen zusammenfällt und deren Radius durch den Betrag der maximalen Abweichung der Soll-Position von der Ist-Position der Struktur vorgegeben ist.

28. Vorrichtung nach Anspruch 26 oder 27,
wobei das Untersuchungsobjekt mit Hilfe des Koordinatenmeßgerätes so positionierbar ist, dass das vom Computer-Tomographen erfaßte Volumen höchstens den x-fachen Rauminhalt der Toleranzkugel bzw. des Toleranzvolumens besitzt, wobei x eine vorgebbare Zahl vorzugsweise größer als 1 ist.

29. Vorrichtung nach einem der Ansprüche 24 bis 28,
wobei die relative Lage und Orientierung des CT-Koordinatensystems gegenüber dem MG-Koordinatensystem vorgegeben sind oder durch Einmessung ermittelbar sind.

30. Vorrichtung nach Anspruch 29, wobei
(i) mittels des Koordinatenmeßgerätes die Lage der mindestens drei ausgewählten Punkte des Untersuchungsobjektes (1) bezüglich des MG-Koordinatensystems bestimmbar ist,
(ii) die auf das MG-Koordinatensystem bezogene Soll-Position der Struktur mit Hilfe der gemäß (i) erzielten Meßergebnisse berechenbar ist, und
(iii) die Soll-Position der Struktur vom MG-Koordinatensystem auf das CT-Koordinatensystem umrechenbar ist, so dass die Lage der Soll-Position im CT-Koordinatensystem bestimmbar ist.

31. Vorrichtung nach Anspruch 30,
wobei das Untersuchungsobjekt (1) gegenüber dem Computer-Tomographen unter Verwendung der gemäß Schritt (iii) erhaltenen, auf das CT-Koordinatensystem bezogenen Soll-Position der Struktur mittels einer Verfahreinrichtung (3) so steuerbar ist, dass sich das Toleranzvolumen und daher auch die Struktur in dem vom Computer-Tomographen erfaßten Volumen befindet.

32. Vorrichtung nach einem der Ansprüche 24 bis 31,
wobei mit Hilfe des Computer-Tomographen ein dreidimensionales digitales CT-Bild des Toleranzvolumens einschließlich der Struktur erstellbar und als CT-Datensatz speicherbar und die Ist-Position der Struktur im CT-Koordinatensystem aus dem CT-Datensatz bestimmbar ist.

33. Vorrichtung nach Anspruch 24, wobei
- der Computer-Tomograph eine Röntgenquelle (5) und einen zweidimensional ortsauflösenden Detektor (6) aufweist, der eine aktive Detektorfläche besitzt, die für die von der Röntgenquelle (5) abgegebene Röntgenstrahlung empfindlich ist,
- das Bildfeld des Computer-Tomographen durch die Größe der aktiven Detektorfläche gegeben ist,
- die Soll-Position der Struktur in Bezug auf mindestens drei ausgewählte, nicht kollineare Punkte des Untersuchungsobjektes (1) vorgegeben ist und die Ist-Position um höchstens eine Toleranzabweichung von der Soll-Position verschieden ist, so dass sich die Ist-Position innerhalb eines z.B. kugelförmigen Toleranzvolumens befindet, dessen Rand um höchstens die Toleranzabweichung von der Soll-Position entfernt ist, und
- die relative Lage und die relative Orientierung des CT-Koordinatensystems gegenüber dem MG-Koordinatensystem bekannt oder durch Einmessung ermittelbar sind,
wobei
a) mittels des Koordinatenmeßgerätes die Lage der mindestens drei ausgewählten Punkte des Untersuchungsobjektes (1) bezüglich des MG-Koordinatensystems bestimmbar ist,
b) hieraus die auf das MG-Koordinatensystem bezogene Soll-Position der Struktur berechenbar ist,
c) die Soll-Position der Struktur vom MG-Koordinatensystem auf das CT-Koordinatensystem umrechenbar ist, so dass die Lage derselben im CT-Koordinatensystem bestimmbar ist,
d) die Relativposition des Untersuchungsobjektes (1) bezüglich des Computer-Tomographen unter Verwendung der auf das CT-Koordinatensystem bezogenen Soll-Position der Struktur mittels einer Verfahreinrichtung (3) so steuerbar ist, dass sich das Toleranzvolumen und daher auch die Struktur in demjenigen Volumen befindet, welches der Computer-Tomograph zu erfassen imstande ist, und
e) der Computer-Tomograph imstande ist, ein dreidimensionales digitales CT-Bild des Toleranzvolumens einschließlich der Struktur zu erstellen und als CT-Datensatz zu speichern,
so dass die Ist-Position sowie die Form der Struktur im CT-Koordinatensystem aus dem CT-Datensatz bestimmbar sind.

34. Vorrichtung nach Anspruch 33,
wobei das Toleranzvolumen eine Toleranzkugel ist, so dass deren Radius durch die Toleranzabweichung und deren Mittelpunkt durch die Soll-Position gegeben ist.

35. Vorrichtung nach Anspruch 33 oder 34,
wobei der Computer-Tomograph so steuerbar ist, dass sich das Zentrum des Toleranzvolumens im wesentlichen im Zentrum des von dem Computer-Tomographen erfaßbaren Volumens befindet.

36. Vorrichtung nach einem der Ansprüche 33 bis 35,
wobei der Computer-Tomograph so steuerbar ist, dass bei zentrischer Projektion des Toleranzvolumens mit der Röntgenquelle (5) als Projektionszentrum das Bildfeld durch die Projektion des Toleranzvolumens auf den Detektor vollständig ausgefüllt ist.

37. Vorrichtung nach einem der Ansprüche 33 bis 35,
wobei der Computer-Tomograph so steuerbar ist, dass bei zentrischer Projektion des Toleranzvolumens mit der Röntgenquelle (5) als Projektionszentrum
- der kleinste Durchmesser der Projektion des Toleranzvolumens auf den Detektor und der kleinste Durchmesser des Bildfeldes des Computer-Tomographen im wesentlichen gleich groß sind, oder
- der größte Durchmesser der Projektion des Toleranzvolumens auf den Detektor und der größte Durchmesser des Bildfeldes des Computer-Tomographen im wesentlichen gleich groß sind, oder
- der größte Durchmesser der Projektion des Toleranzvolumens auf den Detektor und der kleinste Durchmesser des Bildfeldes des Computer-Tomographen im wesentlichen gleich groß sind.

## Claims

1. A process for determining the actual position of a structure of an object to be examined (1) in a coordinate system, whereby a CT scanner is employed which uses CT technology, having a first coordinate system, the CT coordinate system, related to said CT scanner, **and a coordinate measuring instrument (Ml) is employed which is** either a tactile or an optical coordinate measuring instrument or a multisensor coordinate measuring instrument or an ultrasonic coordinate measuring instrument, having a second coordinate system, the MI coordinate system, related to said coordinate measuring instrument, wherein
a) the coordinates of the object to be examined (1) are determined **in the Ml coordinate system,**
b) a target position of the structure within the object to be examined (1) is predefined,
c) after the execution of steps a) and b), the target position is determined in the MI coordinate system,
d) and, using the result of step c), the object to be examined (1) is positioned in such a way that the target position of the structure comes to lie within the volume detected by the CT scanner,
or
aa) the coordinates of the object to be examined (1) are determined in the CT coordinate system,
bb) a target position of the structure within the object to be examined (1) is predefined,
cc) after the execution of steps aa) and bb), the target position is determined in the CT coordinate system,
dd) and, using the result of step cc), the object to be examined (1) is positioned in such a way that the target position of the structure comes to lie within the area that can be detected by the coordinate measuring instrument.

2. The process according to Claim 1, wherein,
in the case of a predefined target position of the structure, relative to at least three selected, non-co-linear points of the object to be examined (1), the object to be examined is positioned using the coordinate measuring instrument in such a way that at least a part of the object to be examined (1) lies within the volume detected by the CT scanner and this part of the object to be examined (1) contains the target position of the structure.

3. The process according to Claim 1 or 2, wherein,
at a predefined maximum deviation of the target position from the actual position of the structure of the object to be examined (1), said object is positioned using the coordinate measuring instrument in such a way that the target position as well as the actual position of the structure lie within the volume detected by the CT scanner.

4. The process according to Claim 3, wherein,
- the actual position differs from the target position by a predefined tolerance deviation at the most, so that the actual position lies within a tolerance volume whose edge is at a distance from the target position by the tolerance deviation at the most, and
- the object to be examined is positioned using the coordinate measuring instrument in such a way that the tolerance volume lies completely within the volume detected by the CT scanner.

5. The process according to Claim 4, wherein
the tolerance volume is a sphere, a tolerance sphere, whose mid-point coincides with the target positions and whose radius is predefined by the amount of the maximum deviation of the target position from the actual position of the structure.

6. The process according to Claim 4 or 5, wherein
the object to be examined is positioned using the coordinate measuring instrument in such a way that the volume detected by the CT scanner has, at the most, the x-fold volume of the tolerance sphere or of the tolerance volume, whereby x is a predefinable number that is preferably greater than 1.

7. The process according to Claim 1, wherein
- in the case of a predefined target position of the structure, relative to at least three selected, non-co-linear points of the object to be examined (1), the object to be examined (1) is positioned using the CT scanner in such a way that at least a part of the object to be examined (1) lies within the area that can be detected by the coordinate measuring instrument and this part of the object to be examined (1) contains the target position of the structure,
- at a predefined maximum deviation of the target position from the actual position of the structure of the object to be examined (1), said object is positioned using the CT scanner in such a way that the target position as well as the actual position of the structure lie within the area that can be detected by the coordinate measuring instrument,
- the actual position differs from the target position by a predefined tolerance deviation at the most, so that the actual position lies within a tolerance area whose edge is at a distance from the target position by the tolerance deviation at the most,
- the object to be examined (1) is positioned using the CT scanner in such a way that the tolerance area lies completely within the area that can be detected by the coordinate measuring instrument.

8. The process according to any of Claims 1 to 7, wherein
the relative location and the relative orientation of the CT coordinate system relative to the MI coordinate system are predefined or can be determined by means of calibration.

9. The process according to Claim 2 and 8, wherein,
(i) by means of the coordinate measuring instrument, the location of the at least three selected points of the object to be examined (1) is determined relative to the MI coordinate system,
(ii) the target position of the structure relative to the MI coordinate system is calculated using the measured results obtained in step (i), and
(iii) the target position of the structure is converted from the MI coordinate system to the CT coordinate system so that subsequently the location of the target position in the CT coordinate system is known.

10. The process according to Claim 9, wherein
the object to be examined (1) is positioned relative to the CT scanner by means of a traveling mechanism (3), using the target position of the structure obtained by means of step (iii) with respect to the CT coordinate system, in such a way that the tolerance volume and thus also the structure lie within the volume detected by the CT scanner.

11. The process according to any of Claims 1 to 10, wherein,
using the CT scanner, a three-dimensional digital CT image of the tolerance volume, including the structure, is created and stored as a CT data record, and the actual position of the structure is determined in the CT coordinate system on the basis of the CT data record.

12. The process according to Claim 7, wherein
(i) by means of the CT scanner, the location of the at least three selected points of the object to be examined (1) is determined relative to the CT coordinate system,
(ii) the target position of the structure relative to the CT coordinate system is calculated using the measured results obtained in step (i),
(iii) the target position of the structure is converted from the CT coordinate system to the MI coordinate system so that subsequently the location of the target position in the CT coordinate system is known,
(iv) the object to be examined (1) is positioned relative to the coordinate measuring instrument by means of a traveling mechanism (3), using the target position of the structure obtained by means of step (iii) with respect to the MI coordinate system, in such a way that the tolerance volume and thus also the structure lie within the area that can be detected by the coordinate measuring instrument, and
(v) using the coordinate measuring instrument, a three-dimensional digital image of the tolerance area, including the structure, is created and stored as an MI data record, and the actual position of the structure is determined in the MI coordinate system on the basis of the MI data record.

13. The process according to Claim 1, wherein,
- the CT scanner used is one that has an X-ray source (5) and a two-dimensional, position-resolving detector (6) having an active detector surface that is sensitive to the radiation emitted by the X-ray source (5), whereby the image field of the CT scanner is defined by the size of the active detector surface,
- the target position of the structure, relative to at least three selected, non-co-linear points of the object to be examined (1), is predefined, and the actual position differs from the target position by a tolerance deviation at the most, so that the actual position lies within a, for example, spherical tolerance volume whose edge is at a distance from the target position by the tolerance deviation at the most, and
- the relative location and the relative orientation of the CT coordinate system relative to the MI coordinate system are known or are determined by means of calibration,
and the following steps are carried out:
a) by means of the coordinate measuring instrument, the location of the at least three selected points of the object to be examined (1) are determined relative to the MI coordinate system,
b) the target position of the structure relative to the MI coordinate system is calculated using the measured results obtained in step a),
c) the target position of the structure is converted from the MI coordinate system to the CT coordinate system, so that the location thereof in the CT coordinate system is known,
d) the relative position of the object to be examined (1) is regulated with respect to the CT scanner by means of a traveling mechanism (3), using the target position of the structure obtained by means of step c) relative to the CT coordinate system, in such a way that the tolerance volume and thus also the structure lie within the volume that can be detected by the CT scanner,
e) by means of the CT scanner, a three-dimensional digital CT image of the tolerance volume, including the structure, is created and stored as a CT data record, and
f) the actual position of the structure is determined in the CT coordinate system on the basis of the CT data record.

14. The process according to Claim 13, wherein
the tolerance volume is a tolerance sphere, so that its radius is defined by the tolerance deviation and its mid-point is defined by the target position.

15. The process according to Claim 13 or 14, wherein
the CT scanner in process step d) is regulated in such a way that the center of the tolerance volume is essentially located in the center of the volume that can be detected by the CT scanner.

16. The process according to any of Claims 13 to 15, wherein
the CT scanner is regulated in such a way that, with the centered projection of the tolerance volume with the X-ray source (5) as the center of projection, the image field is completely filled by the projection of the tolerance volume onto the detector.

17. The process according to Claim 15 or 16, wherein
the CT scanner is regulated in such a way that, with the centered projection of the tolerance volume with the X-ray source (5) as the center of projection
- the smallest diameter of the projection of the tolerance volume onto the detector and the smallest diameter of the image field of the CT scanner are essentially equal in size, or
- the largest diameter of the projection of the tolerance volume onto the detector and the largest diameter of the image field of the CT scanner are essentially equal in size, or
- the largest diameter of the projection of the tolerance volume onto the detector and the smallest diameter of the image field of the CT scanner are essentially equal in size.

18. The process according to any of Claims 1 to 17, wherein,
in addition to the location of the structure, the shape of the structure is also determined on the basis of the CT image or the CT data record.

19. The process according to any of Claims 1 to 17, wherein
the shape of the structure rather than the location of the structure is determined on the basis of the CT image or the CT data record.

20. The process according to any of Claims 1 to 19, wherein
the position of at least three, preferably at least four, selected space points of a calibration object is determined with the CT scanner in the CT coordinate system as well as with the coordinate measuring instrument in the MI coordinate system, and the comparison of the results thus obtained makes it possible to determine the relative location and the relative orientation of the CT coordinate system relative to the MI coordinate system.

21. The process according to Claim 20, wherein
the object to be examined (1) and the calibration object are identical.

22. The process according to any of Claims 1 to 21, wherein,
A) the object to be examined (1) is rotated incrementally around an axis of rotation in order to create the CT image,
B) for each of the rotational positions that the object to be examined (1) thus passes through, a two-dimensional transmission X-ray image of the object to be examined (1) is taken with the detector (6), and
C) the three-dimensional CT image is created on the basis of the two-dimensional transmission X-ray images thus obtained.

23. The process according to Claim 22, wherein,
D) after steps A) and B) have been carried out, the object to be examined (1) is shifted translatorily by a certain distance, preferably in a direction parallel to the axis of rotation, and then once again rotated incrementally around the axis of rotation;
E) for each of the rotational positions that the object to be examined passes through in step D), a two-dimensional transmission X-ray image of the object to be examined (1) is once again taken with the detector (6), and
F) another three-dimensional CT image is created on the basis of the two-dimensional transmission X-ray image obtained in step E).

24. A device for determining the actual position of a structure of an object to be examined (1) in a coordinate system,
- with a CT scanner having a first coordinate system, the CT coordinate system, related to said CT scanner,
- and with a coordinate measuring instrument which is either a tactile or an optical coordinate measuring instrument or a multisensor coordinate measuring instrument or an ultrasonic coordinate measuring instrument, having a second coordinate system, the MI coordinate system, related to said coordinate measuring instrument,
whereby the coordinates of the object to be examined (1) can be determined in the MI coordinate system, and a target position of the structure within the object to be examined(1) is predefined, so that
- the target position can be determined in the MI coordinate system,
- and in the case of a predefined target position of the structure, relative to at least three selected, non-co-linear points of the object to be examined (1), the object to be examined can be positioned using the coordinate measuring instrument in such a way that at least a part of the object to be examined (1) lies within the volume detected by the CT scanner and this part of the object to be examined (1) contains the target position of the structure, as a result of which the target position of the structure comes to lie within the volume detected by the CT scanner,
whereby the CT scanner and the multisensor coordinate measuring instrument are integrated into one single device.

25. The device according to Claim 24, wherein,
at a predefined maximum deviation of the target position from the actual position of the structure of the object to be examined (1), said object can be positioned using the coordinate measuring instrument in such a way that the target position as well as the actual position of the structure lie within the volume detected by the CT scanner.

26. The device according to Claim 25, wherein,
- the actual position differs from the target position by a predefined tolerance deviation at the most, so that the actual position lies within a tolerance volume whose edge is at a distance from the target position by the tolerance deviation at the most, and
- the object to be examined can be positioned using the coordinate measuring instrument in such a way that the tolerance volume lies completely within the volume detected by the CT scanner.

27. The device according to Claim 26, wherein
the tolerance volume is a sphere, a tolerance sphere, whose mid-point coincides with the target positions and whose radius is predefined by the amount of the maximum deviation of the target position from the actual position of the structure.

28. The device according to Claim 26 or 27, wherein
the object to be examined can be positioned using the coordinate measuring instrument in such a way that the volume detected by the CT scanner has, at the most, the x-fold volume of the tolerance sphere or of the tolerance volume, whereby x is a predefinable number that is preferably greater than 1.

29. The device according to any of Claims 24 to 28, wherein
the relative location and the relative orientation of the CT coordinate system relative to the MI coordinate system are predefined or can be determined by means of calibration.

30. The device according to Claim 29, wherein,
(i) by means of the coordinate measuring instrument, the location of the at least three selected points of the object to be examined (1) can be determined relative to the MI coordinate system,
(ii) the target position of the structure relative to the MI coordinate system can be calculated using the measured results obtained in step (i), and
(iii) the target position of the structure can be converted from the MI coordinate system to the CT coordinate system so that the location of the target position can be determined in the CT coordinate system.

31. The device according to Claim 30, wherein
the object to be examined (1) can be positioned relative to the CT scanner by means of a traveling mechanism (3), using the target position of the structure obtained by means of step (iii) with respect to the CT coordinate system, in such a way that the tolerance volume and thus also the structure lie within the volume detected by the CT scanner.

32. The device according to any of Claims 24 to 31, wherein,
using the CT scanner, a three-dimensional digital CT image of the tolerance volume, including the structure, can be created and stored as a CT data record, and the actual position of the structure in the CT coordinate system can be determined on the basis of the CT data record.

33. The device according to Claim 24, wherein,
- the CT scanner has an X-ray source (5) and a two-dimensional position-resolving detector (6) having an active detector surface that is sensitive to the radiation emitted by the X-ray source (5),
- the image field of the CT scanner is defined by the size of the active detector surface,
- the target position of the structure, relative to at least three selected, non-co-linear points of the object to be examined (1), is predefined and the actual position differs from the target position by a tolerance deviation at the most, so that the actual position lies within a, for example, spherical tolerance volume whose edge is at a distance from the target position by the tolerance deviation at the most, and
- the relative location and the relative orientation of the CT coordinate system relative to the MI coordinate system are known or can be determined by means of calibration,
whereby
a) by means of the coordinate measuring instrument, the location of the at least three selected points of the object to be examined (1) can be determined relative to the MI coordinate system,
b) the target position of the structure relative to the MI coordinate system can be calculated from this,
c) the target position of the structure can be converted from the MI coordinate system to the CT coordinate system, so that the location thereof can be determined in the CT coordinate system,
d) the relative position of the object to be examined (1) relative to the CT scanner can be regulated by means of a traveling mechanism (3), using the target position of the structure relative to the CT coordinate system, in such a way that the tolerance volume and thus also the structure lie within the volume that can be detected by the CT scanner, and
e) the CT scanner can create a three-dimensional digital CT image of the tolerance volume, including the structure, and can store it as a CT data record,
so that the actual position as well as the shape of the structure can be determined in the CT coordinate system on the basis of the CT data record.

34. The device according to Claim 33, wherein
the tolerance volume is a tolerance sphere, so that its radius is defined by the tolerance deviation and its mid-point is defined by the target position.

35. The device according to Claim 33 or 34, wherein
the CT scanner can be regulated in such a way that the center of the tolerance volume is located essentially in the center of the volume that can be detected by the CT scanner.

36. The device according to any of Claims 33 to 35, wherein
the CT scanner can be regulated in such a way that, with the centered projection of the tolerance volume with the X-ray source (5) as the center of projection, the image field is completely filled by the projection of the tolerance volume onto the detector.

37. The device according to any of Claims 33 to 35, wherein
the CT scanner can be regulated in such a way that, with the centered projection of the tolerance volume with the X-ray source (5) as the center of projection,
- the smallest diameter of the projection of the tolerance volume onto the detector and the smallest diameter of the image field of the CT scanner are essentially equal in size, or
- the largest diameter of the projection of the tolerance volume onto the detector and the largest diameter of the image field of the CT scanner are essentially equal in size, or
- the largest diameter of the projection of the tolerance volume onto the detector and the smallest diameter of the image field of the CT scanner are essentially equal in size.

## Revendications

1. Procédé pour la détermination de la position instantanée d'une structure d'un objet à examiner (1) dans un système de coordonnées, utilisant un tomographe par ordinateur (CT) faisant appel à la technique CT avec un premier système de coordonnées rapporté au tomographe par ordinateur, le système des coordonnées CT, et un appareil de mesure des coordonnées qui est, soit un appareil de mesure des coordonnées tactil ou optique, soit un appareil de mesure des coordonnées par capteurs multiples, soit un appareil de mesure des coordonnées par ultrasons, avec un deuxième système de coordonnées rapporté audit appareil de mesure, le système des coordonnées de l'appareil de mesure ou système des coordonnées AM,
a) les coordonnées de l'objet à examiner (1) étant déterminées dans le système des coordonnées AM,
b) une position théorique de la structure à l'intérieur de l'objet à examiner (1) étant prédéfinie,
c) la position théorique étant déterminée dans le système des coordonnées AM après exécution des étapes a) et b),
d) et, en utilisant le résultat de l'étape c), l'objet à examiner (1) étant positionné de sorte que la position théorique de la structure vient se situer à l'intérieur du volume saisi par le tomographe par ordinateur
ou
aa) les coordonnées de l'objet à examiner (1) étant déterminées dans le système des coordonnées CT,
bb) une position théorique de la structure à l'intérieur de l'objet à examiner (1) étant prédéfinie,
cc) la position théorique étant déterminée dans le système des coordonnées CT après exécution des étapes aa) et bb),
dd) et, en utilisant le résultat de l'étape cc), l'objet à examiner (1) étant positionné de sorte que la position théorique de la structure vient se situer à l'intérieur de la zone pouvant être saisie par l'appareil de mesure des coordonnées.

2. Procédé selon la revendication 1, dans lequel
l'objet à examiner (1) est positionné à l'aide de l'appareil de mesure des coordonnées, étant donné une position théorique de la structure prédéfinie par rapport à au moins trois points non colinéaires sélectionnés de l'objet à examiner (1), de sorte que au moins une partie de l'objet à examiner (1) se situe dans le volume saisi par le tomographe par ordinateur et cette partie de l'objet à examiner (1) contient la position théorique de la structure.

3. Procédé selon la revendication 1 ou 2, dans lequel
l'objet à examiner (1) est positionné à l'aide de l'appareil de mesure des coordonnées, étant donné un écart maximal prédéfini entre la position théorique et la position instantanée de la structure de l'objet à examiner (1), de sorte que aussi bien la position théorique que la position instantanée de la structure se situent dans le volume saisi par le tomographe par ordinateur.

4. Procédé selon la revendication 3, dans lequel
- la position instantanée diffère de la position théorique de tout au plus un écart de tolérance prédéfini, de sorte que la position instantanée se trouve à l'intérieur d'un volume de tolérance dont le bord n'est pas plus éloigné de la position théorique que l'écart de tolérance, et
- l'objet à examiner (1) estt positionné à l'aide de l'appareil de mesure des coordonnées de sorte que le volume de tolérance se situe complètement dans le volume saisi par le tomographe par ordinateur.

5. Procédé selon la revendication 4, dans lequel
le volume de tolérance est une sphère, la sphère de tolérance, dont le centre coïncide avec la position théorique et dont le rayon est donné par le montant de l'écart maximum entre la position théorique et la position instantanée de la structure.

6. Procédé selon la revendication 4 ou 5, dans lequel
l'objet à examiner (1) est positionné à l'aide de l'appareil de mesure des coordonnées, de sorte que le volume saisi par le tomographe par ordinateur possède tout au plus le volume de la sphère de tolérance ou du volume de tolérance multiplié par x, x étant un nombre prédéfinissable, de préférence supérieur à 1.

7. Procédé selon la revendication 1, dans lequel
- l'objet à examiner (1) est positionné à l'aide du tomographe par ordinateur, étant donnée une position théorique prédéfinie de la structure par rapport à au moins trois points non colinéaires sélectionnés de l'objet à examiner (1), de sorte que au moins une partie de l'objet à examiner (1) se situe dans la zone saisie par l'appareil de mesure des coordonnées et cette partie de l'objet à examiner (1) contient la position théorique de la structure,
- l'objet à examiner (1) est positionné à l'aide du tomographe par ordinateur, étant donné un écart maximal prédéfini entre la position théorique et la position instantanée de la structure de l'objet à examiner (1), de sorte que aussi bien la position théorique que la position instantanée de la structure se situent dans la zone pouvant être saisie par l'appareil de mesure des coordonnées,
- la position instantanée diffère de la position théorique de tout au plus un écart de tolérance prédéfini, de sorte que la position instantanée se trouve à l'intérieur d'une zone de tolérance dont le bord n'est pas plus éloigné de la position théorique que l'écart de tolérance, et
- l'objet à examiner (1) est positionné à l'aide du tomographe par ordinateur de sorte que la zone de tolérance se situe complètement dans la zone saisie par l'appareil de mesure des coordonnées.

8. Procédé selon l'une des revendications 1 à 7, dans lequel
la position relative et l'orientation relative du système des coordonnées CT sont prédéfinies par rapport au système des coordonnées AM ou sont déterminées par mesure.

9. Procédé selon les revendications 2 et 8, dans lequel
(i) la position de au moins trois points sélectionnés de l'objet à examiner (1) est déterminée par rapport au système des coordonnées AM au moyen de l'appareil de mesure des coordonnées,
(ii) la position théorique de la structure rapportée au système des coordonnées AM est calculée au moyen des résultats de mesure obtenus à l'étape (i), et
(iii) la position théorique de la structure est convertie du système des coordonnées AM dans le système des coordonnées CT, si bien qu'ensuite, la position de la position théorique est connue dans le système des coordonnées CT.

10. Procédé selon la revendication 9, dans lequel
l'objet à examiner (1) est piloté par rapport au tomographe par ordinateur, en utilisant la position théorique de la structure rapportée au système des coordonnées CT obtenue selon l'étape (iii), au moyen d'un dispositif de déplacement (3), de sorte que le volume de tolérance et de ce fait aussi la structure se trouve dans le volume saisi par le tomographe par ordinateur.

11. Procédé selon l'une des revendications 1 à 10, dans lequel
une image CT tridimensionnelle digitale du volume de tolérance y compris de la structure est effectuée à l'aide du tomographe par ordinateur et mémorisée en tant qu'ensemble de données CT et la position instantanée de la structure est déterminée à partir de l'ensemble de données CT dans le système des coordonnées CT.

12. Procédé selon la revendication 7, dans lequel
(i) la position de au moins trois points sélectionnés de l'objet à examiner (1) est déterminée par rapport au système des coordonnées CT au moyen du tomographe par ordinateur,
(ii) la position théorique de la structure rapportée au système des coordonnées CT est calculée au moyen des résultats de mesure obtenus à l'étape (i),
(iii) la position théorique de la structure est convertie du système des coordonnées CT dans le système des coordonnées AM, si bien qu'ensuite, la position de la structure est connue dans le système des coordonnées AM.
(iv) l'objet à examiner (1) est piloté par rapport à l'appareil de mesure des coordonnées, en utilisant la position théorique de la structure rapportée au système des coordonnées AM obtenue selon l'étape (iii), au moyen d'un dispositif de déplacement (3), de sorte que le volume de tolérance et de ce fait aussi la structure se trouve dans la zone saisie par l'appareil de mesure des coordonnées, et
(v) une image tridimensionnelle digitale de la zone de tolérance y compris de la structure est effectuée à l'aide de l'appareil de mesure des coordonnées et mémorisée en tant qu'ensemble de données AM et la position instantanée de la structure est déterminée dans le système des coordonnées AM à partir de l'ensemble de données AM.

13. Procédé selon la revendication 1, dans lequel
- le tomographe par ordinateur utilisé possède une source de rayons X (5) et un détecteur à résolution locale 2D (6) qui possède une surface de détection active sensible au rayonnement X émis par la source de rayons X (5), le champ d'image du tomographe par ordinateur étant donné par la dimension de la surface de détection active,
- la position théorique de la structure par rapport à au moins trois points non colinéaires de l'objet à examiner (1) sélectionnés est prédéfinie et la position instantanée diffère de la position théorique de tout au plus un écart de tolérance, de sorte que la position instantanée se trouve à l'intérieur d'un volume de tolérance, par exemple en forme de sphère, dont le bord n'est pas plus éloigné de la position théorique que l'écart de tolérance, et
- la position relative et l'orientation relative du système des coordonnées CT par rapport au système des coordonnées AM sont connues ou sont déterminées par mesure,
et les étapes suivantes sont exécutées :
a) la position de au moins trois points sélectionnés de l'objet à examiner (1) par rapport au système des coordonnées AM est déterminée au moyen de l'appareil de mesure des coordonnées,
b) la position théorique de la structure rapportée au système des coordonnées AM est calculée à l'aide des résultats de mesure obtenus à l'étape a),
c) la position théorique de la structure est convertie du système des coordonnées AM dans le système des coordonnées CT, de sorte que la position de ladite structure est connue dans le système des coordonnées CT,
d) la position relative de l'objet à examiner (1) par rapport au tomographe par ordinateur est pilotée, en utilisant la position théorique de la structure rapportée au système des coordonnées CT obtenue selon l'étape c), au moyen d'un dispositif de déplacement (3), de sorte que le volume de tolérance et de ce fait aussi la structure se trouve dans le volume que le tomographe par ordinateur est capable de saisir,
e) une image CT tridimensionnelle digitale du volume de tolérance y compris de la structure est effectuée à l'aide du tomographe par ordinateur et mémorisée en tant qu'ensemble de données CT, et
f) la position instantanée de la structure dans le système des coordonnées CT est déterminée à partir de l'ensemble de données CT.

14. Procédé selon la revendication 13, dans lequel
le volume de tolérance est une sphère de tolérance, en sorte que le rayon est donné par l'écart de tolérance et le point central par la position théorique.

15. Procédé selon la revendication 13 ou 14, dans lequel
le tomographe par ordinateur est piloté dans l'étape opératoire d) de sorte que le centre du volume de tolérance se trouve essentiellement au centre du volume pouvant être saisi par le tomographe par ordinateur.

16. Procédé selon l'une des revendications 13 à 15, dans lequel
le tomographe par ordinateur est piloté de sorte que, étant donné une projection centrée du volume de tolérance avec la source de rayons X (5) comme centre de projection, le champ d'image est complètement rempli par la projection du volume de tolérance sur le détecteur.

17. Procédé selon l'une des revendications 15 ou 16, dans lequel
le tomographe par ordinateur est piloté de sorte que, étant donné une projection centrée du volume de tolérance avec la source de rayons X (5) en tant que centre de projection,
- le diamètre minimal de la projection du volume de tolérance sur le détecteur et le diamètre minimal du champ d'image du tomographe par ordinateur ont pratiquement la même dimension, ou
- le diamètre maximal de la projection du volume de tolérance sur le détecteur et le diamètre maximal du champ d'image du tomographe par ordinateur ont pratiquement la même dimension, ou
- le diamètre maximal de la projection du volume de tolérance sur le détecteur et le diamètre minimal du champ d'image du tomographe par ordinateur ont pratiquement la même dimension.

18. Procédé selon l'une des revendications 1 à 17, dans lequel
l'image CT ou l'ensemble de données CT permet de déterminer également la forme de la structure, en plus de la position de la structure.

19. Procédé selon l'une des revendications 1 à 17, dans lequel
l'image CT ou l'ensemble de données du CT permet de déterminer la forme de la structure au lieu de la position de la structure.

20. Procédé selon l'une des revendications 1 à 19, dans lequel
la position d'au moins trois, de préférence quatre des points spatiaux sélectionnés d'un objet de calibrage est déterminée aussi bien avec le tomographe par ordinateur dans le système des coordonnées CT qu'avec un appareil de mesure des coordonnées dans le système des coordonnées AM et la position relative et l'orientation relative du système des coordonnées CT sont déterminées par rapport au système des coordonnées AM à partir de la comparaison des résultats ainsi obtenus.

21. Procédé selon la revendication 20, dans lequel
l'objet à examiner (1) et l'objet de calibrage sont identiques.

22. Procédé selon l'une des revendications 1 à 21, dans lequel
A) l'objet à examiner (1) tourne progressivement autour d'un axe de rotation pour effectuer l'image CT,
B) uen radiographie 2D de l'objet à examiner (1) est prise par le détecteur (6) pour chacune des positions de rotation par lesquelles passe l'objet à examiner (1), et
C) une image CT tridimensionnelle est effectuée à partir des radiographies 2D ainsi obtenus.

23. Procédé selon la revendication 22, dans lequel
D) l'objet à examiner (1) est déplacé d'une certaine distance dans un mouvement de translation, de préférence dans un sens parallèle à l'axe de rotation, après exécution des étapes A) et B), et tourne ensuite à nouveau progressivement autour de l'axe de rotation ;
E) une radiographie 2D de l'objet à examiner (1) est prise à son tour par le détecteur (6) pour chacune des positions de rotation par lesquelles passe l'objet à examiner (1) dans l'étape D), et
F) une nouvelle image CT tridimensionnelle est effectuée à partir des radiographies 2D obtenues à l'étape E).

24. Dispositif pour la détermination de la position instantanée d'une structure d'un objet à examiner (1) dans un système de coordonnées, comprenant
- un tomographe par ordinateur (CT) avec un premier système de coordonnées rapporté au tomographe par ordinateur, le système des coordonnées CT,
- et un appareil de mesure des coordonnées qui est soit un appareil de mesure des coordonnées tactil ou optique, soit un appareil de mesure des coordonnées par capteurs multiples, soit un appareil de mesure des coordonnées par ultrasons, avec un deuxième système de coordonnées rapporté à ce dernier, le système des coordonnées AM,
les coordonnées de l'objet à examiner (1) pouvant être déterminées dans le système des coordonnées AM et une position théorique de la structure étant prédéfinie à l'intérieur de l'objet à examiner (1), de sorte que
- la position théorique peut être déterminée dans le système des coordonnées AM
- et l'objet à examiner (1) pouvant être positionné à l'aide de l'appareil de mesure des coordonnées étant donné une position théorique de la structure prédéfinie par rapport à au moins trois points non colinéaires sélectionnés de l'objet à examiner (1), de sorte que au moins une partie de l'objet à examiner (1) se situe dans le volume saisi par le tomographe par ordinateur et cette partie de l'objet à examiner (1) contient la position théorique de la structure, ce par quoi la position théorique de la structure vient à se situer à l'intérieur du volume saisi par le tomographe par ordinateur,
le tomographe par ordinateur et l'appareil de mesure des coordonnées par capteurs multiples étant intégrés dans un seul dispositif.

25. Dispositif selon la revendication 24, dans lequel
l'objet à examiner (1) peut être positionné à l'aide de l'appareil de mesure des coordonnées, étant donné un écart maximal prédéfini entre la position théorique et la position instantanée de la structure de l'objet à examiner (1), de sorte que aussi bien la position théorique que la position instantanée de la structure se situent dans le volume saisi par le tomographe par ordinateur.

26. Dispositif selon la revendication 25, dans lequel
- la position instantanée diffère de la position théorique de tout au plus un écart de tolérance prédéfini, de sorte que la position instantanée se trouve à l'intérieur d'un volume de tolérance dont le bord n'est pas plus éloigné de la position théorique que l'écart de tolérance, et
- l'objet à examiner (1) peut être positionné à l'aide de l'appareil de mesure des coordonnées, de sorte que le volume de tolérance se situe complètement dans le volume saisi par le tomographe par ordinateur.

27. Dispositif selon la revendication 26, dans lequel
le volume de tolérance est une sphère, la sphère de tolérance, dont le centre coïncide avec la position théorique et dont le rayon est donné par le montant de l'écart maximum entre la position théorique et la position instantanée de la structure.

28. Dispositif selon la revendication 26 ou 27, dans lequel
l'objet à examiner (1) peut être positionné à l'aide de l'appareil de mesure des coordonnées de sorte que le volume saisi par le tomographe par ordinateur possède tout au plus le volume de la sphère de tolérance ou du volume de tolérance multiplié par x, x étant un nombre prédéfinissable, de préférence supérieur à 1.

29. Dispositif selon l'une des revendications 24 à 28, dans lequel
la position relative et l'orientation relative du système des coordonnées CT sont prédéfinies par rapport au système des coordonnées AM ou peuvent être déterminées par mesure.

30. Dispositif selon la revendication 29, dans lequel
(i) la position de au moins trois points sélectionnés de l'objet à examiner (1) peut être déterminée par rapport au système des coordonnées AM au moyen de l'appareil de mesure des coordonnées,
(ii) la position théorique de la structure rapportée au système des coordonnées AM peut être calculée au moyen des résultats de mesure obtenus à l'étape (i), et
(iii) la position théorique de la structure peut être convertie du système des coordonnées AM dans le système des coordonnées CT, si bien qu'ensuite, la position de la structure est connue dans le système des coordonnées CT.

31. Dispositif selon la revendication 30, dans lequel
l'objet à examiner (1) peut être piloté par rapport au tomographe par ordinateur, en utilisant la position théorique de la structure rapportée au système des coordonnées CT obtenue selon l'étape (iii), au moyen d'un dispositif de déplacement (3), de sorte que le volume de tolérance et de ce fait aussi la structure se trouve dans le volume saisi par le tomographe par ordinateur.

32. Dispositif selon l'une des revendications 24 à 31, dans lequel
une image CT tridimensionnelle digitale du volume de tolérance y compris de la structure peut être effectuée à l'aide du tomographe par ordinateur et être mémorisée en tant qu'ensemble de données CT et la position instantanée de la structure peut être déterminée dans le système de coordonnées CT à partir de l'ensemble de données CT.

33. Dispositif selon la revendication 24, dans lequel
- le tomographe par ordinateur présente une source de rayons X (5) et un détecteur à résolution locale 2D (6) qui possède une surface de détection active, laquelle est sensible au rayonnement X émis par la source de rayons X (5),
- le champ d'image du tomographe par ordinateur est donné par la dimension de la surface de détection active,
- la position théorique de la structure par rapport à au moins trois points non colinéaires de l'objet à examiner (1) sélectionnés est prédéfinie et la position instantanée diffère de la position théorique de tout au plus un écart de tolérance, de sorte que la position instantanée se trouve à l'intérieur d'un volume de tolérance, par exemple en forme de sphère, dont le bord n'est pas plus éloigné de la position théorique que l'écart de tolérance, et
- la position relative et l'orientation relative du système des coordonnées CT par rapport au système des coordonnées AM sont connues ou peuvent être déterminées par mesure,
et dans lequel
a) la position de au moins trois points sélectionnés de l'objet à examiner (1) par rapport au système des coordonnées AM peuvent être déterminée au moyen de l'appareil de mesure des coordonnées,
b) la position théorique de la structure rapportée au système des coordonnées AM peut être calculée à l'aide des résultats de mesure obtenus à l'étape a),
c) la position théorique de la structure peut être convertie du système des coordonnées AM dans le système des coordonnées CT, de sorte que la position de ladite structure peut être déterminée dans le système des coordonnées CT,
d) la position relative de l'objet à examiner (1) par rapport au tomographe par ordinateur peut être pilotée, en utilisant la position théorique de la structure rapportée au système des coordonnées CT, au moyen d'un dispositif de déplacement (3), de sorte que le volume de tolérance et de ce fait aussi la structure se trouve dans le volume que le tomographe par ordinateur est capable de saisir,
e) le tomographe par ordinateur est en mesure d'établir une image CT tridimensionnelle digitale du volume de tolérance y compris de la structure et de les mémoriser sous forme d'un ensemble de données CT,
de sorte que la position instantanée ainse que la forme de la structure peuvent être déterminées dans le système de coordonnées CT à partir de l'ensemble de données CT.

34. Dispositif selon la revendication 33, dans lequel
le volume de tolérance est une sphère de tolérance, en sorte que son rayon est donné par l'écart de tolérance et son point central par la position théorique.

35. Dispositif selon la revendication 33 ou 34, dans lequel
le tomographe par ordinateur peut être piloté de sorte que le centre du volume de tolérance se trouve essentiellement au centre du volume pouvant être saisi par le tomographe par ordinateur.

36. Dispositif selon l'une des revendications 33 à 35, dans lequel
le tomographe par ordinateur peut être piloté de sorte que, étant donné une projection centrée du volume de tolérance avec la source de rayons X (5) comme centre de projection, le champ d'image est complètement rempli par la projection du volume de tolérance sur le détecteur.

37. Dispositif selon l'une des revendications 33 à 35, dans lequel
le tomographe par ordinateur peut être piloté de sorte que, étant donné une projection centrée du volume de tolérance avec la source de rayons X (5) en tant que centre de projection,
- le diamètre minimal de la projection du volume de tolérance sur le détecteur et le diamètre minimal du champ d'image du tomographe par ordinateur ont pratiquement la même dimension, ou
- le diamètre maximal de la projection du volume de tolérance sur le détecteur et le diamètre maximal du champ d'image du tomographe par ordinateur ont pratiquement la même dimension, ou
- le diamètre maximal de la projection du volume de tolérance sur le détecteur et le diamètre minimal du champ d'image du tomographe par ordinateur ont pratiquement la même dimension.
